# EUROPEAN PATENT APPLICATION

(11) **EP 4 840 970 A1**
(43) Date of publication of application: **18.11.2026**
(21) Application number: 25382489.0
(22) Date of filing: 15.05.2025
(51) Int. Cl.: C12Q 1/6883

(54) **BIOMARKERS OF CEREBELLAR ATROPHY**

(71) Applicant: Hospital Sant Joan de Déu, 08950 Esplugues de Llobregat Barcelona (ES); Flomics Biotech, S.L., 08005 Barcelona (ES); Fundació Centre de Regulació Genòmica, 08003 Barcelona (ES)
(72) Inventor: SERRANO GIMARÉ, Mercedes, 08840 VILADECANS (ES); HERNANDO DAVALILLO, Cristina, 08021 BARCELONA (ES); CABÚS FORNAS, Lluc, 08005 BARCELONA (ES); BELMONTE BALLESTER, Sonia, 08005 BARCELONA (ES); PÉREZ BOZA, Jennifer, 08005 BARCELONA (ES); OLIVIER WEBER, Marc, 08005 BARCELONA (ES); LAGARDE, Julien, 08005 BARCELONA (ES); FERREIRA CURADO E SILVA, João Filipe, 08005 BARCELONA (ES); AIRES COUTINHO GUEDES, André, 08005 BARCELONA (ES); CARBONELL SALA, Sílvia, 08018 BARCELONA (ES)
(74) Representative: ZBM Patents - Zea, Barlocci & Markvardsen

(57) **Abstract**

The invention is addressed to an *in vitro* method for the diagnosis of cerebellar atrophy in a subject comprising:
(i) determining, in a sample isolated from the subject, the expression level of at least one miRNA selected from the group consisting of miR-122-5p, miR-2110, miR-140-5p, miR-217-5p, miR-140-3p, and combinations thereof,
(ii) calculating a variable S, wherein: $S = A - B - C + D - E$ and wherein:
- A is the expression level of miR-122-5p determined in step (i), or 0 when the expression level of miR-122-5p is not determined in step (i),
- B is the expression level of miR-2110 determined in step (i), or 0 when the expression level of miR-2110 is not determined in step (i),
- C is the expression level of miR-140-5p determined in step (i), or 0 when the expression level of miR-140-5p is not determined in step (i),
- D is the expression level of miR-217-5p determined in step (i), or 0 when the expression level of miR-217-5p is not determined in step (i),
- E is the expression level of miR-140-3p determined in step (i), or 0 when the expression level of miR-140-3p is not determined in step (i), and

(iii) diagnosing the subject as having cerebellar atrophy if S calculated in step (ii) is equal or higher than a corresponding reference value and as not having cerebellar atrophy if S calculated in step (ii) is lower than the corresponding reference value.

The present invention also provides the use of miR-122-5p, miR-2110, miR-140-5p, miR-217-5p, miR-140-3p, and combinations thereof as biomarkers of cerebellar atrophy.

## Description

### Technical Field

The present invention is related to the field of diagnosis. In particular, the present invention provides non-invasive methods for the diagnosis of cerebellar atrophy, based on measuring the amount of specific cell-free RNAs in a bodily fluid sample.

### Background Art

Cerebellar atrophy (CA) refers to a condition characterized by an irreversible loss of tissue of the cerebellum, which results from the loss of neurons (mainly Purkinje cells) in this part of the posterior fossa that ultimately loses normal volume, what can be observed by neuroimaging. Anatomically, the cerebellum from patients with CA shows initially normal structures in a posterior fossa with normal size, which displays enlarged fissures (interfolial spaces) in comparison to the foliae, secondary to loss of tissue. Sometimes a subtle increase in the cortical signal in the magnetic resonance images can be also observed.

Cerebellum corresponds to the area of the encephalon that controls coordination and balance. Not surprisingly, symptoms of CA may include a wide-based, unsteady, lurching walk, often accompanied by a tremor in the trunk of the body; slow, unsteady and jerky movement of the arms or legs; slowed and slurred speech and/or abnormal movements of the eyes (nystagmus, strabismus, saccadic movements or dysmetria). CA has been also associated with cognitive functions, causing abnormalities in cognition, including executive functions, processing speed, language, and various processes related to emotion-related disorders. CA is thus associated to major disabilities that significantly compromises the quality of life of the affected patients.

CA can be caused by very different factors, including non-genetic and genetic factors (due to inherited mutations or spontaneous mutations). One example of disorder caused by genetic factors that is associated with CA is the Phosphomannomutase deficiency (PMM2-CDG), the most frequent congenital disorder of N-linked glycosylation. PMM2-CDG is caused by mutations in the PMM2 gene, encoding an enzyme that catalyses the second step in the N-glycosylation pathway, the conversion of mannose-6-phosphate to mannose-1-phosphate.

Early clinical signs of PMM2-CDG usually include abnormal fat distribution, inverted nipples, strabismus and hypotonia. Infants develop ataxia, psychomotor delay and extraneurological manifestations, including failure to thrive, enteropathy, hepatic dysfunction, abnormal coagulation and cardiac and renal involvement. The phenotype is extremely variable, and while some patients develop a severe systemic illness leading to early death, milder forms with variable systemic and mild neurological involvement are also detected.

The Central Nervous System (CNS) and peripheral nervous system are the prime systems affected by PMM2-CDG deficiency, although, as in other CDGs, other systems are also affected. Cerebellar dysfunction is a regular condition associated with PMM2-CDG. At birth, most children have marked hypotrophy of the vermis and cerebellar hemispheres, followed by progression towards atrophy. Many key neurological symptoms found in PMM2-CDG patients can be explained by this cerebellar syndrome (ataxia, dysmetria, tremor, abnormal eye movements, dysarthria, but also cognitive deficits or low intelligence quotient (lQ)), leading to major disability. From a patient's perspective, it has been recently reported that, in the long term, the neurological symptoms are those altering the most the quality of life and daily functionality.

Although PMM2-CDG currently has no cure, different strategies of treatment are under development.. However, clinical assessment may be determinant for an appropriate therapeutic approach. Unfortunately, clinical assessments have not standardized evaluation tools, as neurodevelopment may hinder or confound many symptoms, or bias the interpretations of clinical changes. Moreover, evaluations requiring collaboration and understanding of the maneuvers cannot be applied to patients with severe intellectual disabilities or behavioral disturbances. Abnormal sialotransferrin profile is a direct consequence of abnormal N-glycosylation and has been used for the diagnosis. However, it is not useful as a biomarker since the profile may spontaneously improve with age. No reliable molecular biomarkers have thus been described so far..

Therefore, there is a need in the state of the art for a method allowing to accurately identify CA patients, particularly in a non-invasive, time and cost-effective manner.

### Summary of Invention

The authors of the present invention have surprisingly found that different miRNAs or combination of miRNAs selected from the list consisting of miR-122-5p, miR-2110, miR-140-5p, miR-217-5p, and miR-140-3p, show an expression level in blood samples that allows to classify subjects into CA and non-CA subjects, and even to determine if the CA is caused or associated with PMM2-CDG or rather with a non-PMM2-CDG disorder.

As disclosed in the examples below, the authors of the present invention have determined the expression level of miRNAs miR-122-5p, miR-2110, miR-140-5p, miR-217-5p in blood samples from control subjects with no CA, subjects having CA- PMM2-CDG and subjects having CA but not PMM2-CDG (in particular, caused or associated to cerebellar hemorrhage in a premature infant, CACNA1G protein dysfunction, CACNA1A protein dysfunction, CASK protein dysfunction, PPP2R1A protein dysfunction). They have then analyzed the diagnostic potential of these markers for the detection of CA patients, and more particularly to detect CA-PMM2-CDG patients or CA patients not affected by PMM2-CDG, based on a receiver operating characteristic (ROC) curve for each miRNA or combination of miRNAs. As shown in table 1 below, the Area Under the Curve (AUC) thus obtained for several of said miRNAs or combination of miRNAs is higher than 0.7, higher than 0.8 or even almost of 0.9 for the discrimination of CA-PMM2-CDG patients from non-CA subjects. Table 2 also shows that the Area Under the Curve (AUC) obtained for several of said miRNAs or combination of miRNAs is higher than 0.7 for the discrimination of CA patients with no PMM2-CDG from non-CA subjects.

Thus, the authors of the present invention have identified several miRNAs or combinations of miRNAs whose expression level determined in blood samples allows to differentiate subjects suffering CA from those not suffering the disease, and more particularly, to identify if the CA is most probably caused by or associated with PMM2-CDG or not. Since the method can be simply performed analyzing a blood sample, it allows a specific identification of CA patients in an efficient, or even outstanding manner, and with a time and cost-effective approach. Additionally, since the method allows to provide information about the leading cause of CA, it is also particularly useful for determining in a time and cost-effective manner the most accurate medical response to be provided to the patient.

Thus, in a first aspect, the invention is addressed to an *in vitro* method for the diagnosis of cerebellar atrophy in a subject comprising:
(i) determining, in a sample isolated from the patient, the expression level of at least one miRNA selected from the group consisting of miR-122-5p, miR-2110, miR-140-5p, miR-217-5p, miR-140-3p, and combinations thereof,
(ii) calculating a variable S, wherein: $S = A - B - C + D - E$ and wherein:
   - A is the expression level of miR-122-5p determined in step (i), or 0 when the expression level of miR-122-5p is not determined in step (i),
   - B is the expression level of miR-2110 determined in step (i), or 0 when the expression level of miR-2110 is not determined in step (i),
   - C is the expression level of miR-140-5p determined in step (i), or 0 when the expression level of miR-140-5p is not determined in step (i),
   - D is the expression level of miR-217-5p determined in step (i), or 0 when the expression level of miR-217-5p is not determined in step (i),
   - E is the expression level of miR-140-3p determined in step (i), or 0 when the expression level of miR-140-3p is not determined in step (i), and
(iii) diagnosing the subject as having cerebellar atrophy if S calculated in step (ii) is equal or higher than a corresponding reference value and as not having cerebellar atrophy if S calculated in step (ii) is lower than the corresponding reference value.

In a second aspect, the invention is addressed to an *in vitro* method for deciding or recommending whether to initiate a therapeutic intervention in an individual suspicious of having cerebellar atrophy, wherein the method comprises steps (i)-(ii) as defined in the first aspect and (iii) if S calculated in step (ii) is equal or higher than the corresponding reference value, it is indicative that the individual has to start a therapeutic intervention.

In a third aspect, the invention is addressed to an *in vitro* method for determining the efficacy of a therapeutic intervention in a patient already diagnosed with cerebellar atrophy, wherein the method comprises performing steps (i)-(ii) as defined in the first aspect before and after starting the therapeutic intervention, and if S calculated in step (ii) obtained after starting the therapeutic intervention, is lower than S calculated in step (ii) obtained before starting the therapeutic intervention, it is indicative that the therapeutic intervention is effective in the treatment of cerebellar atrophy.

In a fourth aspect, the invention is addressed to a method for classifying a subject into a subject cohort, wherein the method comprises steps (i)-(ii) as defined in the first aspect and (iii) classifying the subject into:
- a cohort of subjects with high probability of suffering cerebellar atrophy if S calculated in step (ii) is equal or higher than the corresponding reference value, or
- a cohort of subjects with low probability of suffering cerebellar atrophy if S calculated in step (ii) is lower than the corresponding reference value.

In a fifth aspect, the invention is addressed to the use of at least one miRNA selected from the group consisting of miR-122-5p, miR-2110, miR-217-5p, miR-140-5p, miR-140-3p, and combinations thereof, as a biomarker for the diagnosis of cerebellar atrophy; or as a biomarker for deciding or recommending whether to initiate a therapeutic intervention in an individual suspicious of having cerebellar atrophy; or as a biomarker for determining the efficacy of a therapeutic intervention in a patient already diagnosed with cerebellar atrophy; or as a biomarker for classifying a subject into a cohort of subjects with high probabilities of having cerebellar atrophy or of subjects with low probabilities of having cerebellar atrophy.

In a sixth aspect, the invention is addressed to the use of a kit for the diagnosis of cerebellar atrophy in an individual; or for deciding or recommending whether to initiate a therapeutic intervention in an individual suspicious of having cerebellar atrophy; or for determining the efficacy of a therapeutic intervention in a patient already diagnosed with cerebellar atrophy; or for classifying a subject into a subject cohort of subjects with high probabilities of having cerebellar atrophy or of subjects with low probabilities of having cerebellar atrophy; the kit comprising means for detecting the level of expression of at least one miRNA selected from the list consisting of miR-122-5p, miR-2110, miR-140-5p, miR-217-5p, miR-140-3p, and combinations thereof, and optionally a solid support.

### Detailed description of the invention

All terms as used herein in this application, unless otherwise stated, shall be understood in their ordinary meaning as known in the art. Other more specific definitions for certain terms as used in the present application are as set forth below and are intended to apply uniformly through-out the specification and numbered embodiments unless an otherwise expressly set out definition provides a broader definition.

As used herein, the indefinite articles "a" and "an" are synonymous with "at least one" or "one or more." Unless indicated otherwise, definite articles used herein, such as "the" also include the plural of the noun. For purposes of the present invention, any ranges given include both the lower and the upper end-points of the range. Ranges given, such as concentrations and the like, should be considered approximate, unless specifically stated. The term "about" refers to a deviation of plus/minus 10 %, preferably plus/minus 5 %.

### 1. Methods of the invention

### A. In vitro method for the diagnosis of cerebellar atrophy

In a first aspect, the invention is addressed to an *in vitro* method for the diagnosis of cerebellar atrophy in a subject comprising:
(i) determining, in a sample isolated from the patient, the expression level of at least one miRNA selected from the group consisting of miR-122-5p, miR-2110, miR-140-5p, miR-217-5p, miR-140-3p, and combinations thereof,
(ii) calculating a variable S, wherein: $S = A - B - C + D - E$ and wherein:
   - A is the expression level of miR-122-5p determined in step (i), or 0 when the expression level of miR-122-5p is not determined in step (i),
   - B is the expression level of miR-2110 determined in step (i), or 0 when the expression level of miR-2110 is not determined in step (i),
   - C is the expression level of miR-140-5p determined in step (i), or 0 when the expression level of miR-140-5p is not determined in step (i),
   - D is the expression level of miR-217-5p determined in step (i), or 0 when the expression level of miR-217-5p is not determined in step (i),
   - E is the expression level of miR-140-3p determined in step (i), or 0 when the expression level of miR-140-3p is not determined in step (i), and
(iii) diagnosing the subject as having cerebellar atrophy if S calculated in step (ii) is equal or higher than a corresponding reference value and as not having cerebellar atrophy if S calculated in step (ii) is lower than the corresponding reference value.

The term "diagnose", "diagnosing" or "diagnosis", as used herein, relates to the evaluation of the probability according to which a subject suffers a specific pathology (in this case, suffering from cerebellar atrophy). As the skilled in the art will understand, such evaluation may not be correct for 100% of the subjects to be diagnosed, although it preferably is. The term, however, requires being able to identify a statistically significant part of the subjects. Whether a subject is statistically significant can be determined without further ado by the person skilled in the art using various well known statistical evaluation tools, e.g., determination of confidence intervals, p-value determination, Student's t-test, Mann-Whitney test, etc. Preferred confidence intervals are at least 50%, at least 60%, at least 70%, at least 80%, at least 90%, or at least 95%. The p-values are, particularly, 0.05, 0.01, 0.005 or lower.

The term "cerebellar atrophy" or "CA", as used herein, refers to an atrophy of the cerebellum, the area of the brain that controls coordination and balance, wherein the term "atrophy" refers to an irreversible loss of cells from a tissue or organ. It thus refers to an irreversible loss of cells from the cerebellum. Anatomically, the cerebellum from patients with CA shows initially normal structures in a posterior fossa with normal size, which displays enlarged fissures (interfolial spaces) in comparison to the foliae secondary to loss of tissue.

Symptoms of CA may include a wide-based, unsteady, lurching walk, often accompanied by a tremor in the trunk of the body; slow, unsteady and jerky movement of the arms or legs; slowed and slurred speech and/or rapid, small movements of the eyes (nystagmus).

Diagnostic methods for CA generally include transfontanellar ultrasonography (in infants), a CT (computed tomography) and/or, as gold standard, cranial magnetic resonance imaging (MRI).

CA can be caused by very different factors, including genetic factors, non-genetic factors, or a combination of both, i.e. it can also be multifactorial.

Non-genetic factors that can cause CA include alcoholism and malnutrition. Regarding genetic factors that can cause CA, a common one is Phosphomannomutase 2 (PMM2) deficiency, also known as PMM2-congenital disorder of glycosylation (PMM2-CDG), or congenital disorder of glycosylation type la.

The term "Phosphomannomutase 2 (PMM2) deficiency", "PMM2-congenital disorder of glycosylation", "PMM2-CDG", or "as congenital disorder of glycosylation type la", as used herein, refers to a genetic disease in which a cerebellar atrophy progression is nearly always observed. It is the most frequent congenital disorder of N-linked glycosylation. Cerebellar dysfunction is the main daily functional limitation in PMM2-CDG patients..

PMM2-CDG is a well-defined condition, for which several symptoms have been described, including:
- In children:
   - Clinical findings: Faltering growth, Hypothyroidism, hypogonadism, Esotropia, Pericardial effusion, Abnormal subcutaneous fat pattern including increased suprapubic fat pad, skin dimpling, and inverted nipples or subcutaneous fat pads having a toughened, puffy, or uneven consistency, Seizures, Stroke-like episodes, Recurrent infections with concerns about immunologic dysfunction and vaccine non-responsiveness, Osteopenia, scoliosis, Cerebellar hypoplasia/atrophy and small brain stem and additional characteristic findings on brain MRI (See Clinical Description.)
   - Laboratory findings: Hepatic dysfunction (elevated transaminases), Coagulopathy with abnormal prothrombin time, low serum concentration of factors IX and XI, antithrombin III, protein C, and/or protein S, Occasional hypoglycemia seen in infants, with some due to hyperinsulinemia, Possible hypothyroidism reflected by elevated TSH; however, thyroxine levels, performed by equilibrium dialysis, are the most reliable marker for thyroid function, Osteopenia frequently with normal calcium, magnesium, and phosphate levels, Proteinuria and aminoaciduria with elevated creatinine reported in some individuals
- In an adolescent or adult.
   - Clinical findings: Cerebellar dysfunction (ataxia, dysarthria, dysmetria) and characteristic findings on brain MRI (See Clinical Description.), Non-progressive cognitive impairment, Seizures, Stroke-like episodes, Peripheral neuropathy with or without muscle wasting, Absent or atypical secondary sexual development in females, small testes in males, Retinitis pigmentosa and myopia, Progressive scoliosis with truncal shortening, Joint contractures
   - Laboratory findings: Elevated liver function tests (transaminases), Low serum concentration of factors IX and XI, antithrombin III, protein C, and/or protein S, Gonadal dysfunction in most but not all adult females with varying levels of FSH, LH, and estradiol. Most males have normal pubescence but may have low testosterone and sex hormone-binding globulin.

The wide variety of signs and symptoms in PMM2-CDG are likely due to the production of abnormally glycosylated proteins in many organs and tissues. Commonly used methods for the diagnosis of PMM2-CDG generally requires a first identification of the clinical symptoms of the subject (as those described just above), proband with type I transferrin isoform analysis and identification of either biallelic pathogenic variants in *PMM2* on molecular genetic testing or (if results of molecular genetic testing are uncertain) low levels of phosphomannomutase (PMM) enzyme activity, following well-known techniques by an expert in the field.

PMM2-CDG is caused by mutations in the PMM2 gene that reduce the activity of the PMM2 enzyme, which catalyses the second step in the N-glycosylation pathway, the conversion of mannose-6-phosphate to mannose-1-phosphate. PMM2-CDG is inherited in an autosomal recessive manner. Several pathogenic PMM2 protein variants found in PMM2-CDG patients have been described, being the most common ones the R141H and F119L amino acid sequence variants (see for instance Briso-Montiano A. et al. "Insight on molecular pathogenesis and pharmacochaperoning potential in phosphomannomutase 2 deficiency, provided by novel human phosphomannomutase 2 structures". J Inherit Metab Dis. 2022;45(2):318-333). In a particular embodiment the R141H PMM2 protein variant corresponds to the protein encoded by PMM2 gene comprising the mutation disclosed in NCBI (CLINVAR) database, accession number VCV000007706.120 (as of date Septembre, 16, 2024). In another particular embodiment the F141L PMM2 protein variant corresponds to the protein encoded by PMM2 gene comprising the mutation disclosed in NCBI (CLINVAR) database, accession number VCV000371231.2 (as of date December 24, 2022), or encoded by PMM2 gene comprising the mutation disclosed in NCBI (CLINVAR) database, accession number VCV000007711.59 (as of date August 25, 2024). Another common pathogenic PMM2 variant found in PMM2-CDG patients, particularly in Spanish PMM2-CDG patients, is the p.Thr237Met variant (abbreviated T237M) also described in Briso-Montiano A. et al. 2022. In a particular embodiment, the T237M PMM2 protein variant corresponds to the protein encoded by PMM2 gene comprising the mutation disclosed in NCBI (CLINVAR) database, accession number VCV000021145.62 (as of date Septembre 8, 2024). Additional PMM2 pathogenic variants found in PMM2-CDG patients are provided in Briso-Montiano A. et al. 2022, particularly in section "3.2 Structure based analysis of hPMM2 pathogenic variants", more particularly in Fig. 5, yet more particularly in Fig. 3, of Briso A. et al. 2022. PMM2 protein variants or PMM2 gene mutants found in PMM2-CDG patients are also disclosed in OMIM database with accession number 601785, as of date March 17, 2022.,

In a particular embodiment, the term CA, as used herein, refers to CA caused by, or associated with PMM2-CDG, particularly caused by PMM2-CDG. In another particular embodiment, the term "CA caused by or associated with PMM2-CDG", as used herein, refers to CA caused by or associated with, particularly, caused by, a PMM2 protein dysfunction. More particularly, it refers to CA caused by or associated with, particularly caused by, the expression of a pathogenic PMM2 protein variant, particularly wherein said PMM2 pathogenic protein variant is any of those described in the definition of "PMM2-CDG" above. In another particular embodiment, the term "CA caused by or associated with PMM2-CDG", as used herein, refers to CA caused by or associated with, particularly caused by, a PMM2 gene mutation selected from those described in the definition of "PMM2-CDG" above. In another particular embodiment, it refers to CA caused by or associated with, particularly caused by, the expression of a pathogenic PMM2 protein variant, particularly wherein said PMM2 pathogenic protein variant is the protein encoded by any of the PMM2 gene mutants described in the definition of "PMM2-CDG" above.

In another particular embodiment, the term CA, as used herein, refers to CA caused by, or associated with PMM2 protein dysfunction. Particularly, the term "PMM2 protein dysfunction" as used herein, is interchangeable in any of the aspects of the invention with "the expression of a pathogenic PMM2 protein variant" selected from those provided in the definition of "PMM2-CDG", or with "at least one mutation in the PMM2 encoding gene" selected from any of those provided in the definition of PMM2-CDG, or with "the expression of a mutant of the PMM2 encoding gene" selected from any of those provided in the definition of PMM2. The term "at least one" as used herein, particularly refers to one, two, or three, more particularly to one

The term "Phosphomannomutase 2 protein" or "PMM2 protein", as used herein, refers to the protein encoded by gene PMM2 with NCBI accession number NG_009209.1 as of date March 25, 2023. Concomitantly, the term "PMM2 gene", as used herein, refers to the gene with NCBI accession number NG_009209.1 as of date March 25, 2023

Other non-limiting examples of congenital diseases, or genetic factors, that cause CA include diseases caused by or associated with, particularly caused by, CACNA1G protein dysfunction, CACNA1A protein dysfunction, CASK protein dysfunction, and/or PPP2R1A protein dysfunction.

Thus, in a particular embodiment, the term CA, as used herein, refers to CA with a cause different than PMM2-CDG or not associated with PMM2-CDG (i.e. with a cause different than PMM2 protein dysfunction, or not associated to PMM2 protein dysfunction). More particularly, the term CA, as used herein, refers to CA caused by or associated with, particularly caused by, CACNA1G protein dysfunction, CACNA1A protein dysfunction, CASK protein dysfunction, or PPP2R1A protein dysfunction. In another particular embodiment, the term CA, as used herein, refers to CA caused by or associated with, particularly caused by protein dysfunction in a protein encoded by at least one gene selected from the list consisting of CACNA1G, CACA1A, CASK, PPP2R1A and combinations thereof. In a more particular embodiment, the term CA, as used herein, refers to CA caused by or associated with, particularly caused by protein dysfunction in a protein encoded by a gene selected from the list consisting of CACNA1G, CACA1A, CASK, and PP2R1A.

The term "protein dysfunction" as used herein, refers to a characteristic of a protein consisting in that the function of the protein is altered, as compared to that of the corresponding wild-type protein. In the context of the invention, the term "function of the protein" in the definition of "protein dysfunction", particularly refers to the function of the corresponding protein (i.e. PMM2, CACNA1G, CACNA1A, CASK, and/or PPP2R1A) directly or indirectly involved in cerebellum activity, particularly in the development of the cerebellum or in the structure maintenance of the cerebellum. The expression "function of the protein is altered", as used in the definition of "protein dysfunction", envisages both the option that the corresponding function of the protein is increased with respect to the that of the corresponding wild-type protein, and the option that the corresponding function of the protein is decreased with respect to that of the corresponding wild-type protein. In any case, such protein dysfunction, in the context of the present invention, is understood to contribute to the development of CA. Protein dysfunction, as used herein, thus refers to a loss-of-function or a gain-of-function of the corresponding protein, as compared to that of the wild-type protein, as long as the expression of the said dysfunctional protein in a subject negatively affects the function or structure of the cerebellum of the subject, particularly, ultimately leads to the development of CA as defined herein in the subject.

Protein dysfunction usually results from at least one mutation in the gene encoding the protein, so that the protein cannot exert its function adequately, or as the corresponding wild-type protein. As well known by an expert in the field, a mutation or nucleotide sequence modification typically falls into one or more of three classes: substitution, insertion, or deletion . The term "wild-type-protein", as used herein, refers to the term well-understood by a skilled person. For instance, in the case of proteins PMM2, CACNA1G, CACNA1A, CASK, and PPP2R1A the corresponding wild-type-protein is that falling within the definition provided herein for PMM2, CACNA1G, CACNA1A, CASK, and PPP2R1A proteins, respectively. Concomitantly, the wild-type gene also refers to the term well-understood by a skilled person and in the case of genes PMM2, CACNA1G, CACNA1A, CASK, and PPP2R1A, the term refers to the gene falling within the definition provided herein for PMM2, CACNA1G, CACNA1A, CASK, and PPP2R1A genes, respectively (i.e. see the definition of the term "PMM2 potein" and "PMM2 gene" above, the definition of CACNA1G, CACNA1A, CASK, and PPP2R1A proteins and genes below)..

The term "CACNA1G protein", as used herein, refers to the protein encoded by gene CACN1G with NCBI accession number NG_032024.1, as of date August 24, 2020. Concomitantly, the term "CACN1G gene" refers to the gene with NCBI accession number NG_032024.1, as of date August 24, 2020.

The term "CACNA1A protein" as used herein, refers to the protein encoded by the gene CACNA1A with NCBI accession number NG_011569.1, as of date October 15, 2024. Concomitantly, the term "CACNA1A gene" refers to the gene with NCBI accession number NG_011569.1, as of date October 15, 2024.

The term "CASK protein" as used herein, refers to the protein encoded by the gene CASK with NCBI accession number NG_016754.2, as of date December 08, 2024. Concomitantly, the term "CASK gene" refers to the gene with NCBI accession number NG_016754.2, as of date December 08, 2024.

The term "PPP2R1A protein" as used herein, refers to the protein encoded by the gene PPP2R1A with NCBI accession number NG_047068.2, as of date October 06, 2024. Concomitantly, the term "PPP2R1A gene" refers to the gene with NCBI accession number NG_047068.2 as of date October 06, 2024.

In a particular embodiment, the term "protein dysfunction" refers to a protein gain-of-function, particularly a gain-of-function as compared to the corresponding wild-type protein. A protein with a gain-of-function, as referred herein, generally results from one or several mutations in the gene encoding the protein, that overall lead to a protein with a gain-of-function as compared to that of the corresponding wild-type protein, and that negatively affects the function or structure of the cerebellum of the subject, and particularly, ultimately leads to the development of CA as defined herein. Thus, a protein with a gain-of-function is generally encoded by a gene comprising at least one gain-of-function mutation, particularly one gain-of-function mutation, wherein said mutation negatively affects the function or structure of the cerebellum, and particularly, ultimately leads to the development of CA as defined herein.

In another particular embodiment, the term "protein dysfunction" as used herein, refers to a protein loss-of-function, particularly a loss-of-function as compared to that of the corresponding wild-type protein. A protein with a loss-of-function, as referred herein, generally results from one or several mutations in the gene encoding the protein, that overall lead to a protein with a loss-of-function as compared to that of the corresponding wild-type protein, and that negatively affects the function or structure of the cerebellum of the subject, and particularly, ultimately leads to the development of CA as defined herein. Thus, a protein with a loss-of-function is generally encoded by a gene comprising at least one loss-of-function mutation, particularly one loss-of-function mutation, wherein said mutation negatively affects the function or structure of the cerebellum, and particularly, ultimately leads to the development of CA as defined herein.

In a particular embodiment, the term CA, as used herein, refers to CA caused by or associated with, particularly caused by, CACNA1G protein dysfunction (i.e. protein dysfunction of the protein encoded by gene CACNA1G). In a particular embodiment, CACNA1G protein dysfunction refers to a gain- or loss-of-function of CACNA1G protein, particularly a gain-of-function of CACNA1G protein. In a more particular embodiment, CACNA1G protein dysfunction refers to a protein dysfunction caused by a mutation in the CACNA1G encoding gene selected from those disclosed in OMIM database, with entry number 604065 (as of date 14 August 2018), particularly from those disclosed therein to lead to affect the activity or structure of the cerebellum, more particularly to lead to CA, yet more particularly to lead to Spinocerebellar ataxia type 42 (SCA42) or Early-onset severe spinocerebellar ataxia 42 (SCA42ND). In a more particular embodiment, CACNA1G protein dysfunction refers to a protein dysfunction caused by a mutant of the CACNA1G encoding gene comprising or consisting of the mutation disclosed in NCBI (CLINVAR) database, accession number VCV000280269.9 (as of date 17 June, 2024). In a yet more more particular embodiment, CACNA1G protein dysfunction refers to a protein dysfunction caused by a mutant of the CACNA1G encoding gene comprising or consisting of that disclosed in NCBI (CLINVAR) database, accession number VCV000280269.9 (as of date 17 June, 2024). In another more particular embodiment, CACNA1G protein dysfunction refers to a protein dysfunction caused by a mutant of the CACNA1G encoding gene comprising or consisting of the mutation disclosed in NCBI (CLINVAR) database, accession number VCV000559581.2 (as of date 24 December, 2022). In a yet more more particular embodiment, CACNA1G protein dysfunction refers to a protein dysfunction caused by a mutant of the CACNA1G encoding gene comprising or consisting of that disclosed in NCBI (CLINVAR) database, accession number VCV000559581.2 (as of date 24 December 2022).

In another particular embodiment, CACNA1G protein dysfunction refers to a disease selected from Spinocerebellar ataxia type 42 (SCA42) or Early-onset severe spinocerebellar ataxia 42 (SCA42ND). In a more particular embodiment, SCA42 and SCA42ND as referred herein, are caused by any of the mutations of the CACNA1G gene indicated above, particularly by the mutation disclosed in NCBI (CLINVAR) database, accession number VCV000280269.9 (as of date 17 June, 2024). In another particular embodiment, SCA42 and SCA42ND as referred herein, are caused by the mutation disclosed in NCBI (CLINVAR) database, accession number VCV000559581.2 (as of date 24 December, 2022).

In a particular embodiment, the term "CACNA1G protein dysfunction" is thus interchangeable in any aspect of the invention with "at least one mutation in the CACNA1G encoding gene" (or with "at least on amino acid modification in the CACNA1G protein") selected from any of those provided herein for said gene (or protein), or with "the expression of a mutant of the CACNA1G encoding gene" selected from any of those provided herein for said gene, or with the "expression of a pathogenic CACNA1G protein variant", particularly wherein said CACNA1G pathogenic protein variant is the protein encoded by any of the CACNA1G gene mutants described herein for CACNA1G gene. The term "at least one" as used herein, particularly refers to one, two, or three, more particularly to one.

In another particular embodiment, CACNA1A protein dysfunction refers to a gain- or loss-of-function of CACNA1A protein. In a more particular embodiment, CACNA1A protein dysfunction refers to a protein dysfunction caused by a mutation in the CACNA1A gene selected from those disclosed in OMIM database, with entry number 601011 (as of date 25 September 2022), particularly those disclosed therein to lead to affect the activity or structure of the cerebellum, more particularly to lead to Episodic Ataxia Type 2, Spinocerebellar Ataxia 6, or progressive cerebellar ataxia, yet more particularly to CA. In a particular embodiment, the term "CACNA1A protein dysfunction" is thus interchangeable in any aspect of the invention with "at least one mutation in the CACNA1A encoding gene" (i.e. or with "at least on amino acid modification in the CACNA1A protein") selected from any of those provided herein for said gene (or protein), or with "the expression of a mutant of the CACNA1A encoding gene " selected m any of those provided herein for said gene, or with the "expression of a pathogenic CACNA1A protein variant", particularly wherein said CACNA1A pathogenic protein variant is the protein encoded by any of the CACNA1A gene mutants described herein for CACNA1A gene.. **The** term "at least one" as used herein, particularly refers to one, two, or three, more particularly to one.

In a particular embodiment, CASK protein dysfunction refers to a gain- or loss-of-function of CASK protein, particularly a loss-of-function of CASK protein, more particularly, a complete knockdown of CASK protein activity. In a more particular embodiment, CASK protein dysfunction refers to a protein dysfunction caused by a mutation in the CASK encoding gene selected from those disclosed in OMIM database, with entry number 300749 (as of date 27 March, 2024), particularly from those disclosed therein to lead to affect the activity or structure of the cerebellum, more particularly to lead to cerebellar hypoplasia, yet more particularly to lead to CA. In a particular embodiment, the term "CASK protein dysfunction" is thus interchangeable in any aspect of the invention with "at least one mutation in the CASK encoding gene" (i.e. or with "art least on amino acid modification in the CASK protein") selected from any of those provided herein for said gene (or protein), or with "the expression of a mutant of the CASK encoding gene in a subject comprising at least one mutation" selected from any of those provided herein for said gene, or with the "expression of a pathogenic CASK protein variant", particularly wherein said CASK pathogenic protein variant is the protein encoded by any of the CASK gene mutants described herein for CASK gene. The term "at least one" as used herein, particularly refers to one, two, or three, more particularly to one.

In a particular embodiment, PPP2R1A protein dysfunction refers to a gain- or loss-of-function of PPP2R1A protein. In a more particular embodiment, PPP2R1A protein dysfunction refers to a protein dysfunction caused by a mutation in the PPP2R1A encoding gene selected from those disclosed in OMIM database, with entry number 605983 (as of date 15 July, 2024), particularly from those disclosed therein to lead to affect the activity or structure of the cerebellum, more particularly to lead to CA. In another more particular embodiment, PPP2R1A protein dysfunction refers to a protein dysfunction caused by a mutation in the PPP2R1A encoding gene selected from those disclosed in Roldán M, Nolasco GA, Armengol L, et al. Advanced Optical Microscopy: Unveiling Functional Insights Regarding a Novel *PPP2R1A* Variant and Its Unreported Phenotype. *Int J Mol Sci.* 2023;24(18):13699. Published 2023 Sep 5 (doi:10.3390/ijms241813699). In a particular embodiment, PPP2R1A protein dysfunction refers to a protein dysfunction caused by the mutation in the PPP2R1A encoding gene disclosed in NCBI (ClinVar) database, accession number VCV002288085.2 (as of date May 1. 2024). In another particular embodiment, PPP2R1A protein dysfunction refers to a protein dysfunction caused by the mutation in the PPP2R1A encoding gene disclosed in NCBI (ClinVar) database, accession number VCV000376506.16 (as of date February 18, 2024). In a particular embodiment, the term "PPP2R1A protein dysfunction" is thus interchangeable in any aspect of the invention with "at least one mutation in the PPP2R1A encoding gene" (i.e. or with "art least on amino acid modification in the PPP2R1A protein") selected from any of those provided herein for said gene (or protein), or with "the expression of a mutant of the PPP2R1A encoding gene in a subject comprising at least one mutation" selected from any of those provided herein for said gene, or with the "expression of a pathogenic PPP2R1A protein variant", particularly wherein said PPP2R1A pathogenic protein variant is the protein encoded by any of the PPP2R1A gene mutants described herein for PPP2R1A gene. The term "at least one" as used herein, particularly refers to one, two, or three, more particularly to one.

A non-limiting example of a multifactorial cause of CA is cerebellar hemorrhage in a premature infant.

The term "cerebellar hemorrhage" or "CbH", as used herein, refers to a type of intracranial hemorrhage, characterized in that bleeding is located in the posterior fossa or cerebellum. It is a common neonatal complication with an ultrasound-detected incidence of 7.6% in extremely preterm infants (gestational age at birth <28 weeks). It has also been reported that between 43% and 75% of the preterm (<32 weeks gestation) infants with "*isolated*" CbH (e.g. absence of supratentorial injury) were severely impaired in cognitive, motor, language and/or behavioral development. On top of extremely preterm birth and other perinatal risk factors, these results suggest that neonatal cerebellar damage make them especially vulnerable for the development of neurodevelopmental defects. The term "premature infant", refers to the term well-known by an expert in the field, i.e. an infant born at preterm, particularly at less than 39, less than 38, less than 38, less than 37, less than 36, less than 35, less than 34, less than 33, less than 32, less than 31, less than 30, less than 29, less than 28, less than 27, less than 26, more particularly less than 32, yet more particularly less than 28, gestation weeks.

In a particular embodiment, the the term CA, as used herein, refers to CA caused by or associated with, particularly caused by, a cerebellar hemorrhage, particularly, cerebellar hemorrhage in a premature infant.

The term "subject" or "individual" or "animal" or "patient" or "mammal" means any subject, particularly a mammalian subject, for whom diagnosis or prognosis is desired. Mammalian subjects include humans, domestic animals, farm animals, and zoo, sports, or pet animals such as dogs, cats, guinea pigs, rabbits, rats, mice, horses, cattle, cows, and so on. In a particular embodiment of the invention, the subject is a mammal. In a more particular embodiment of the invention, the subject is a human. In an even more particular embodiment, the subject is a male or female human of any age or ethnicity. In a particular embodiment, the subject has shown signs to be suffering from CA. Well known signs of a subject to be suffering CA include the symptoms disclosed above in the definition of CA.In a particular embodiment, the miRNAs referred in the present invention are from Homo Sapiens, particularly wherein the subject is human. Thus, in a more particular embodiment, the terms "miR" and "hsa-miR", are interchangeable in the definitions and embodiments of the present invention.

Thus, in a particular embodiment, the subject has been considered to show signs of suffering CA, particularly at least one selected from the list consisting of wide-based, unsteady, lurching walk, a tremor in the trunk of the body; slow, unsteady and jerky movement of the arms or legs; slowed and slurred speech and/or abnormal involuntary movements of the eyes (nystagmus, saccadic movements, strabismus or dysmetria).

The term "sample", or "biological sample", as used herein, refers to biological material isolated from a subject. In a particular embodiment, the sample used in the methods of the invention is isolated from any suitable biological fluid, such as blood, urine, sweat, saliva, or cerebrospinal fluid. In another particular embodiment, the isolated sample used in the methods of the invention is a blood sample, more particularly a plasma or serum sample. In a yet more particular embodiment, the isolated sample used in the methods of the invention is a plasma sample. The sample comprises cell and/or non-cell material of the subject, particularly non-cell material. In the present invention, the sample comprises genetic material, e.g., RNA, miRNA, cell-free RNA, heterogeneous nuclear RNA (hnRNA), mRNA, DNA, genomic DNA (gDNA), complementary DNA (cDNA) etc., from the subject under study. In a particular embodiment, the genetic material is RNA, more particularly cell-free RNA, yet more particularly cell-free miRNA. In a more particular embodiment, the sample comprises RNA, particularly miRNA, but does not comprise DNA. Methods for obtaining samples from a subject, including biofluid, saliva, sweat, blood, plasma or serum samples, are well known to those skilled in the art.

The terms "miRNA" or "microRNA", used interchangeably herein, are endogenous RNAs, some of which are known to regulate the expression of protein-coding genes at the post-transcriptional level. As used herein, the term "miRNA" refers to any type of micro-interfering RNA, including but not limited to, endogenous microRNA and artificial microRNA. Typically, endogenous miRNAs are small RNAs encoded in the genome which are capable of modulating the productive utilization of mRNA. A mature miRNA is a single-stranded RNA molecule of about 21-23 nucleotides in length which is complementary to a target sequence and hybridizes to the target RNA sequence to inhibit its function, particularly its translation. miRNAs themselves are encoded by genes that are transcribed from DNA but not translated into protein (non-coding RNA). Instead, miRNA transcripts are processed from primary transcripts known as pri-miRNA to short stem-loop structures called pre-miRNA and finally to functional/mature miRNA. Mature miRNA molecules are partially complementary to one or more messenger RNA (mRNA) molecules (herein referred to as the target mRNA(s) of a miRNA), which triggers the gene expression down-regulation of the said target mRNA(s), particularly translation inhibition of the said target mRNA(s). Besides their intracellular function, miRNAs can be exported or released by cells into the circulating blood in very stable forms. The term "circulating miRNA" or "C-miRNA", as used herein, also known as "cell-free miRNA" or "cf-miRNA", relates to extracellular miRNA that circulates in the bloodstream.

In a particular embodiment, the miRNAs referred to in the different aspects of the invention are cf-miRNAs.

Pre-miRNAs are referred to as "mir", whereas mature miRNAs are referred to as "miR". This prefix is followed by a dash and a number, the latter often indicating order of naming. miRNAs with nearly identical sequences except for one or two nucleotides are annotated with an additional lower-case letter. Pre-miRNAs that lead to 100% identical mature miRNAs but that are located at different places in the genome are indicated with an additional dash-number suffix. Species of origin is designated with a three-letter prefix.

Some "mir" can have two mature products, one from each arm of the corresponding "mir". For example, miR-122 has two mature products, named miR-122-5p and miR-122-3p. The mature product annotated with 5p arises from the 5 ' arm of the mir-122 hairpin precursor, and the mature product annotated with 3p arises from the 3 'arm of the mir-122 hairpin precursor.

The term "miR-122-5p", as used herein, refers to the miRNA that has SEQ ID NO. 1. In a particular embodiment, the term refers to the mature microRNA from the 5'-end arm of the "mir-122" which has a sequence with accession number NR_029667.1 in the National Center for Biotechnology Information (NCBI) database as of October 1, 2024.

The term "miR-2110", as used herein, refers to the miRNA that has SEQ ID NO. 2. In a particular embodiment, the term refers to the mature microRNA from the 5'-end arm of the "mir-2110" which has a sequence with accession number NR_031747.1 in the National Center for Biotechnology Information (NCBI) database as of November 13, 2024.

The term "miR-140-5p" as used herein, refers to the miRNA that has SEQ ID NO. 3. In a particular embodiment, the term refers to the mature microRNA from the 5'-end arm of the "mir-140" which has a sequence with accession number NR_029681.1 in the National Center for Biotechnology Information (NCBI) database as of November 12, 2024.

The term "miR-217-5p", as used herein, refers to the miRNA that has SEQ ID NO. 4. In a particular embodiment, the term refers to the mature microRNA from the 5'-end arm of the "mir-217" which has a sequence with accession number NR_029630.1 in the National Center for Biotechnology Information (NCBI) database as of October 04, 2024.

The term "miR-140-3p", as used herein, refers to the miRNA that has SEQ ID NO. 5. In a particular embodiment, the term refers to the mature microRNA from the 3'-end arm of the "mir-140" which has a sequence with accession number NR_029681.1 in the National Center for Biotechnology Information (NCBI) database as of November 12, 2024.

The term "expression level of a miRNA", as used herein, refers to the level or amount of the miRNA expressed from its encoding gene, particularly in an indicated sample or unit of biological material. In a particular embodiment, the term "expression level of a miRNA" refers to the level or amount of the pre-miRNA and/or mature miRNA, particularly of the mature miRNA, in the indicated sample or unit of biological material. In a particular embodiment the said sample or unit of biological material corresponds to a certain volume of body fluid comprising this RNA product, or a sample of said body fluid, even more particularly a sample of said body fluid, yet more particularly an isolated blood sample, even yet more particularly an isolated plasma sample.

The level of expression of a miRNA can be provided as a normalized value, an absolute value, a relative value, a value that has an upper and/or a lower limit, a series of values, an average value, a median, a mean value or values expressed by referring to the expression level of a control gene or control miRNA. In a particular embodiment, the expression level of a miRNA is herein provided as a normalized gene expression value. Several methods can be used to normalize the expression level of a miRNA and a normalized expression level of a miRNA can be provided in different units. Non-limiting examples of said units include TPM (Transcripts Per Million, also referred to as Transcripts Per Kilobase Million), RPKM (Reads Per Kilobase Million), FPKM (Fragments Per Kilobase Million) or CPM (Counts Per Million). Methods to obtain the normalized expression level of a miRNA in any of said units are well known by an expert in the field. Particularly, to obtain the normalized expression level of a miRNA in CPM, the following steps are generally followed: 1) The read counts in a sample are determined and this number is divided by 1,000,000, thus obtaining a "per million" scaling factor, 2) The counts obtained for each miRNA is divided by the "per million" scaling factor, thus obtaining the CPM value for each miRNA.

In an embodiment, the expression level of the miRNA or combination of miRNAs used in the methods of the invention is provided as a normalized expression level for each miRNA, particularly in CPM for each miRNA. Thus, in a particular embodiment, the expression level of the miRNA or combination of miRNAs determined in step (i) of the methods of the invention is provided as a normalized expression level, particularly in CPM. As well understood by a skilled person, the units of variable S calculated in step (ii) of the methods of the invention correspond to the units in which the expression level values are provided in step (i). Thus, in a particular embodiment, the units of variable S calculated in step (ii) of the methods of the invention are those of the normalized expression level used in step (i), particularly CPM.

Methods to determine the expression level of a miRNA in a sample are well-known by an expert in the field. Non-limitative examples of such methods include the use of reverse transcription polymerase chain reaction (RT-PCR), wherein extracted RNA is reverse-transcribed, for example, using a GeneAmp^{®} RNA PCR kit (Perkin Elmer, Calif., USA), following the manufacturer's instructions. In some embodiments, miRNA expression levels are determined using a gene expression analysis technology that measures RNA in solution. Examples of such gene expression analysis technologies include, but are not limited to, RNAscope^{™}, RT-PCR, quantitative reverse transcription polymerase chain reaction (RT-qPCR), Nanostring^{®}, QuantiGene^{®}, gNPA^{®}, microarray, use of Next Generation Sequencing (NGS) techniques, or RNA sequence (RNA-seq). RNA sequencing to determine miRNA expression levels can for instance be performed using QIAseq miRNA library kit to obtain miRNA libraries that are then sequenced (for instance with the Illumina^{®} NovaSeq sequencer). The sequencing data obtained can then be analyzed with known bioinformatics tools, such as those described in the examples below, to obtain the expression level of the miRNAs of interest. Thus, in a particular embodiment, gene expression levels are determined in the methods of the invention by methods comprising or consisting of RT-qPCR, RT-PCR, RNA sequencing, or Next Generation Sequencing (NGS) techniques, particularly are determined by methods comprising or consisting of RT-qPCR, RNA sequencing, or Next Generation Sequencing (NGS) techniques. In another embodiment, gene expression levels are determined using a kit for that purpose, such as RNAscope^{™}, Nanostring^{®}, QuantiGene^{®}, gNPA^{®} or a microarray.

In a particular embodiment, the expression level determined in step (i) of the methods of the invention is determined using RNA sequencing techniques, particularly wherein the expression level is provided in CPM. In other particular embodiments, the expression level determined in step (i) of the methods of the invention is determined using RT-qPCR techniques.

In a particular embodiment, the expression level of a miRNA, as used herein, refers to the amount of the RNA transcribed from the gene encoding said miRNA, particularly refers to the amount of the corresponding pre-miRNA, mature miRNA or the combination thereof. More particularly, the expression level of a miRNA, refers to the amount of the corresponding pre-miRNA and mature miRNA. Yet more particularly, the expression level of a miRNA, as used herein, refers to the amount of the corresponding mature miRNA processed from the RNA transcribed from the gene encoding said miRNA.

In the present invention, the term "reference value", or "reference level", as used herein in any of the methods of the invention, is to be understood as a predefined value for variable S for the miRNA or combination of miRNAs whose expression level is determined in step (i) of the methods of the invention. The reference value derives from the expression level in a sample or group of samples, of the miRNA or combination of miRNAs whose expression level is determined in step (i) of the methods of the invention. The samples are taken from a subject or group of subjects wherein the presence, absence, stage, or course of the disease has been properly determined previously. This value is used as a threshold to discriminate subjects wherein the condition to be analyzed is present from those wherein such condition is absent, to determine the stage of the disease, the risk of developing or of being suffering from CA, among others. This reference level is also useful for determining whether the subject has to initiate a medical regimen. In a particular embodiment, the threshold value, as well as the expression level as defined above, can be a normalized value, an absolute value, a relative value, a value that has an upper and/or a lower limit, a series of values, an average value, a median, a mean value or values expressed by referring to the expression level of a control gene or control miRNA. In a more particular embodiment, the threshold value is provided as a range of values. In a particular embodiment of this specific case, a subject is diagnosed as having CA in the method of the first aspect if S calculated in step (ii) of the method is equal or higher than the lower value of a range of values provided as a corresponding reference value, more particularly if S calculated in step (ii) of the method is equal or higher than the higher value of a range of values provided as a corresponding reference value, and as not having CA if S calculated in step (ii) is lower than the lower value of said range of values provided as the corresponding reference value. The same applies to the rest of methods of the invention, with the corresponding conclusion when S is higher than the lower value, particularly higher than the higher value, of the range of values provided as reference values. Said conclusion is that provided in each method of the invention for the cases in which S is higher than the reference value. The same applies as well to the rest of methods of the invention, with the corresponding conclusion when S is lower than the lower value of the range of values provided as reference values. Said conclusion is that provided in each method of the invention for the cases in which S is lower than the reference value.

Methods for obtaining the reference value from the group of subjects selected are well-known in the state of the art (Burtis C. A. et al., 2008, Chapter 14, section "Statistical Treatment of Reference Values"). In a particular case the "reference value" is a cut-off value defined by means of a conventional ROC analysis. As the skilled person will appreciate, optimal cut-off value will be defined according to the particular applications of the diagnostic or prognostic method: purpose, target population for the diagnosis or prognosis, balance between specificity and sensibility, etc. Methods to determine the cut-off value by means of conventional ROC curves are well-known by an expert in the field and include those disclosed in the examples below.

The subject or subjects from whom the "reference value" is obtained may include subject/s wherein the CA is absent, subject/s wherein CA is present, particularly at different stages, or both. The skilled person in the art, making use of the general knowledge, is able to choose the most adequate subject or group of subjects for obtaining the reference level for each of the methods of the present invention. In an embodiment of the present invention, optionally in combination with any of the embodiments provided above or below, the reference value referred in any of the methods of the invention is taken from a group of subjects which do not suffer CA. In one embodiment of the present invention, the reference value referred to in any of the methods of the invention is taken from a group of subjects which have never been diagnosed as suffering CA. In one embodiment of the present invention, the reference value referred to in any of the methods of the invention is taken from a group of subjects which have been diagnosed to not suffer CA. Clinical routine techniques such as transfontanellar ultrasonography (in infants), MRI or TC, can be used to that end.

In one embodiment of the methods of the invention, the reference level is obtained prior to performing the methods of the invention, from the analysis of sets of samples from subjects as just defined above, particularly from a set of samples from subjects determined to not suffer CA, more particularly that have never suffered CA, and from a set of samples from subjects known to suffer CA, particularly at a specific stage or different stages. Particularly, the number of samples in each set can be of at least 10, at least 20, at least 40, at least 44, at least 45, at least 50, at least 60, at least 70, at least 80, at least 90, at least 100, at least 150, at least 200, at least 300, at least 500, at least 750, at at least 1000, at least 5000, or at least 10000. Non-limiting examples of the analysis of both sets of samples to obtain the reference value as defined herein include estimation of distribution algorithms, determination of confidence intervals, computer intensive methods, and machine learning methods, including for instance bagging procedures, boosting procedures, random forest algorithms, regression algorithms and/or combinations thereof. In a particular embodiment, the reference value is defined by means of a conventional ROC analysis of the results obtained, particularly of the results obtained with any of the previous methods, more particularly of the results obtained with machine learning methods, applied to the data obtained from the two sets of samples. In another particular embodiment, the reference value is provided as a range of values, or as a value comprised in a range of reference values, wherein the higher limit of said range of reference values corresponds to the lower value S (as defined above) obtained from the samples of subjects known to have CA as just defined, and the lower limit of the range is the higher value S(as define above) obtained from the samples of subjects determined to not suffer CA.

In a particular embodiment, the units of the reference value correspond to those of variable S as calculated in step (ii) of the methods of the invention. Thus, in a particular embodiment, the reference value used in the methods of the invention is provided in CPM.

The term "higher than a corresponding reference value", as used herein, refers to the fact that the value of variable S calculated in step (ii) of the method for a miRNA or combination of miRNAs is at least about 1%, 2%, 3%, 4%, 5%, 6%, 7%, 8%, 9%, 10%, 12%, 15%, 20%, 25%, 30%, 40%, 50%, 75%, 90%, 95%, 100%, 125%, 150%, 175%, 200%, or more than 200% higher than the value of the reference value for the corresponding miRNA or combination of miRNAs. As well understood by a skilled person, the term "corresponding reference value" as used herein in the methods of the invention, refers to the reference value for the miRNA or group of miRNAs whose expression level is determined in step (i) of the methods of the invention. Thus, in a particular embodiment, when in step (i) the expression level of miR-122-5p and of miR 2110 is determined, the corresponding reference value is that for miR-122-5p and of miR 2110. The term "higher than the higher value of a range of values provided as the corresponding reference value" as used herein, refers to the fact that the value of variable S calculated in step (ii) of the method for a miRNA or combination of miRNAs, is at least about 1%, 2%, 3%, 4%, 5%, 6%, 7%, 8%, 9%, 10%, 12%, 15%, 20%, 25%, 30%, 40%, 50%, 75%, 90%, 95%, 100%, 125%, 150%, 175%, 200% or more than 200% higher than the higher value of a range of values provided as a corresponding reference level. **The** term "higher than the lower value of a range of values provided as the corresponding reference value" as used herein, refers to the fact that the value of variable S calculated in step (ii) of the method for a miRNA or combination of miRNAs, is at least about 1%, 2%, 3%, 4%, 5%, 6%, 7%, 8%, 9%, 10%, 12%, 15%, 20%, 25%, 30%, 40%, 50%, 75%, 90%, 95%, 100%, 125%, 150%, 175%, 200% or more than 200% higher than the lower value of a range of values provided as a corresponding reference level.

**The** term "lower than a corresponding reference value", as used herein, refers to the fact that the value of variable S calculated in step (ii) of the method for a miRNA or combination of miRNAs is at least about 1%, 2%, 3%, 4%, 5%, 6%, 7%, 8%, 9%, 10%, 12%, 15%, 20%, 25%, 30%, 40%, 50%, 75%, 90%, 95%, 100%, 125%, 150%, 175%, 200% or more than 200% lower than the reference value for the corresponding miRNA or combination of miRNAs. **The** term "lower than the lower value of a range of values provided as a corresponding reference value" as used herein, refers to the fact that the value of variable S calculated in step (ii) of the method (for the gene or combination of genes whose expression level is determined in step (i)), is at least about 1%, 2%, 3%, 4%, 5%, 6%, 7%, 8%, 9%, 10%, 12%, 15%, 20%, 25%, 30%, 40%, 50%, 75%, 90%, 95%, 100%, 125%, 150%, 175%, 200% or more than 200% lower than the lower value of a range of values provided as a corresponding reference level.

**The** term "equal to a corresponding reference value", or "equal to a value of a range of reference values", as used herein, refers to the fact that the value of variable S calculated in step (ii) of the method for a miRNA or combination of miRNAs is equal to about 95%, 97%, 98%, 99%, 99.5%, 99.9%, 100%, 101%, 103% 105%, 107% or 110%, particularly to about 98%, the value of the corresponding reference value, or the corresponding value of the range of reference values, respectively.

**The** term "variable S", "S" or "subject's score", as used herein, derives from the expression level of a gene or miRNA or combination of genes and/or miRNAs, particularly of genes or miRNAs, in an isolated sample, and is obtained applying the formula: S= A-B-C+D-E when the expression level of at least one miRNA selected from the group consisting of miR-122-5p, miR-2110, miR-140-5p, miR-217-5p, miR-140-3p, and combinations thereof is determined in step (i), wherein:
- A is the expression level of miR-122-5p when the expression level of miR-122-5p is determined in step (i) of the methods of the invention, or is 0 when the expression level of miR-122-5p is not determined in step (i) of the methods of the invention,
- B is the expression level of miR-2110 when the expression level of miR-2110 is determined in step (i) of the methods of the invention, or is 0 when the expression level of miR-2110 is not determined in step (i) of the methods of the invention,
- C is the expression level of miR-140-5p when the expression level of miR-140-5p is determined in step (i) of the methods of the invention, or is 0 when the expression level of miR-140-5p is not determined in step (i) of the methods of the invention,
- D is the expression level of miR-217-5p when the expression level of miR-217-5p is determined in step (i) of the methods of the invention, or 0 when the expression level of miR-217-5p is not determined in step (i) of the methods of the invention,
- E is the expression level of miR-140-3p when the expression level of miR-140-3p is determined in step (i) of the methods of the invention, or is 0 when the expression level of miR-140-3p is not determined in step (i) of the methods of the invention.

In a particular embodiment, when step (i) further comprises determining the expression level of at least one gene and/or additional miRNA, the variable S is obtained applying the formula:: $S = A - B - C + D - E + {\sum }_{i = 1}^{N} ai Fi ,$ wherein
F is the expression level of the said at least one gene and/or additional miRNA, N is the number of genes and/or additional miRNAs whose expression level is determined in step (i) and a is (1) if an increased expression level of the corresponding gene or miRNA is associated to having cerebellar atrophy or is (-1) if a decreased expression level of the corresponding gene or miRNA is associated to having cerebellar atrophy.

In another particular embodiment, when step (i) of the methods of the invention further comprises determining the expression level of at least one gene or additional miRNA, the variable S is obtained applying the formula:: $S = A - B - C + D - E + {\sum }_{i = 1}^{N} ai Fi ,$ wherein
F is the expression level of the said at least one gene or additional miRNA, N is the number of genes or additional miRNAs whose expression level is determined in step (i) and a is (1) if an increased expression level of the corresponding gene or miRNA is associated to having cerebellar atrophy or is (-1) if a decreased expression level of the corresponding gene or miRNA is associated to having cerebellar atrophy.

In another particular embodiment, when step (i) further comprises determining the expression level of at least one additional miRNA, the variable S is obtained applying the formula: $S = A - B - C + D - E + {\sum }_{i = 1}^{N} ai Fi ,$ wherein
F is the expression level of the said at least one additional miRNA, N is the number of additional miRNAs whose expression level is determined in step (i) and a is (1) if an increased expression level of the corresponding miRNA is associated to having cerebellar atrophy or is (-1) if a decreased expression level of the corresponding miRNA is associated to having cerebellar atrophy.

In other words, S could also be obtained applying the formula $S = A - B - C + D - E + G ,$ wherein:
- A, B, C, D and E are as defined above;
   and:
   - G is obtained applying formula $G = {Σ}^{N} ai Fi$, when step (i) further comprises determining the expression level of at least one gene and/or additional miRNA, particularly of at least one gene or additional miRNA, more particularly when step (i) further comprises determining the expression level of at least one additional miRNA; wherein:

- F, N and a are as defined in the previous paragraphs;
   Or
   - G is 0 when step (i) does not further comprise determining the expression level of at least one gene and/or additional miRNA.

The first aspect of the invention can thus also be worded as an *in vitro* method for the diagnosis of cerebellar atrophy in a subject comprising:
(i) determining, in a sample isolated from the subject, the expression level of at least one miRNA selected from the group consisting of miR-122-5p, miR-2110, miR-140-5p, miR-217-5p, miR-140-3p, and combinations thereof,
(ii) calculating a variable S, wherein: $S = A - B - C + D - E + G ,$ and wherein:
   - A is the expression level of miR-122-5p determined in step (i), or 0 when the expression level of miR-122-5p is not determined in step (i),
   - B is the expression level of miR-2110 determined in step (i), or 0 when the expression level of miR-2110 is not determined in step (i),
   - C is the expression level of miR-140-5p determined in step (i), or 0 when the expression level of miR-140-5p is not determined in step (i),
   - D is the expression level of miR-217-5p determined in step (i), or 0 when the expression level of miR-217-5p is not determined in step (i),
   - E is the expression level of miR-140-3p determined in step (i), or 0 when the expression level of miR-140-3p is not determined in step (i),
   - G is obtained applying formula $G = {\sum }_{i = 1}^{N} ai Fi$, when step (i) further comprises determining the expression level of at least one gene and/or additional miRNA, particularly of at least one gene or additional miRNA, more particularly when step (i) further comprises determining the expression level of at least one additional miRNA; wherein:
      - F is the expression level of the said at least one gene and/or additional miRNA,
      - N is the number of genes and/or additional miRNAs whose expression level is determined in step (i) and
      - a is (1) if an increased expression level of the corresponding gene or miRNA is associated to having cerebellar atrophy or is (-1) if a decreased expression level of the corresponding gene or miRNA is associated to having cerebellar atrophy Or
   - G is 0 when step (i) does not further comprise determining the expression level of at least one gene and/or additional miRNA, and
(iii) diagnosing the subject as having cerebellar atrophy if S calculated in step (ii) is equal or higher than a corresponding reference value and as not having cerebellar atrophy if S calculated in step (ii) is lower than the corresponding reference value.

As well understood by a skilled person, the definition of "expression level" provided above in connection with the expression level of a miRNA, also applies to the expression level of a gene, by substituting the term "miRNA" by "gene" in the definition and embodiments herein.

In an embodiment, CA referred in any of the embodiments of the methods of the invention, is a CA caused by or associated with, particularly caused by, PMM2-CDG, cerebellar hemorrhage in a premature infant, CACNA1G protein dysfunction, CACNA1A protein dysfunction, CASK protein dysfunction, PPP2R1A protein dysfunction or combinations thereof.

In a particular embodiment, CA referred in any of the embodiments of the methods of the invention, is a CA caused by or associated with, particularly caused by, cerebellar hemorrhage in a premature infant, CACNA1G protein dysfunction, CACNA1A protein dysfunction, CASK protein dysfunction, PPP2R1A protein dysfunction or combinations thereof, particularly not caused by nor associated with PMM2-CDG.

In a yet more particular embodiment, CA referred to in any of the embodiments of the methods of the invention, is a CA caused by or associated with, particularly caused by, PMM2-CDG.

In a particular embodiment, CA referred in any of the embodiments of the methods of the invention is a CA caused by or associated with, particularly caused by, cerebellar hemorrhage in a premature infant, particularly wherein it is not caused by nor associated with PMM2-CDG. In another particular embodiment, CA referred in any of the embodiments of the methods of the invention is a CA caused by or associated with, particularly caused by, CACNA1G protein dysfunction, particularly wherein it is not caused by nor associated with PMM2-CDG. In another particular embodiment, CA referred in any of the embodiments of the methods of the invention is a CA caused by or associated with, particularly caused by, CASK protein dysfunction, particularly wherein it is not caused by nor associated with PMM2-CDG. In another particular embodiment, CA referred in any of the embodiments of the methods of the invention is a CA caused by or associated with PPP2R1A protein dysfunction, particularly wherein it is not caused by nor associated with PMM2-CDG.

In another embodiment, when CA is indicated to be a CA not caused by nor associated with PMM2-CDG in any of the embodiments of the methods of the invention, the CA is caused by or associated with, particularly caused by, cerebellar hemorrhage in a premature infant, CACNA1G protein dysfunction, CACNA1A protein dysfunction, CASK protein dysfunction, PPP2R1A protein dysfunction, or a combination thereof. In another particular embodiment, when CA is indicated to be a CA not caused by nor associated with PMM2-CDG in any of the embodiments of the methods of the invention, the CA is caused by or associated with, particularly caused by, hemorrhage in a premature infant. In another particular embodiment, when CA is indicated to be a CA not caused by nor associated with PMM2-CDG in any of the embodiments of the methods of the invention, the CA is caused by or associated with, particularly caused by, CACNA1G protein dysfunction. In another particular embodiment, when CA is indicated to be a CA not caused by nor associated with PMM2-CDG in any of the embodiments of the methods of the invention, the CA is caused by or associated with, particularly caused by, CACNA1A protein dysfunction. In another particular embodiment, when CA is indicated to be a CA not caused by nor associated with PMM2-CDG in any of the embodiments of the methods of the invention, the CA is caused by or associated with, particularly caused by, CASK protein dysfunction. In another particular embodiment, when CA is indicated to be a CA not caused by nor associated with PMM2-CDG in any of embodiments of the methods of the invention, the CA is caused by or associated with, particularly caused by, PPP2R1A protein dysfunction.

In a particular embodiment, PMM2-CDG is as indicated in the definition of PMM2-CDG, more particularly as indicated in any embodiment above addressed to PMM2-CDG.

In a particular embodiment, CACNA1G protein dysfunction is as indicated above in the defition of protein dysfunction, more particularly as indicated in any embodiment above addressed to CACNA1G protein dysfunction.

In a particular embodiment, CACNA1A protein dysfunction is as indicated above in the definition of protein dysfunction, more particularly as indicated in any embodiment above addressed to CACNA1A protein dysfunction.

In a particular embodiment, CASK protein dysfunction is as indicated above in the definition of protein dysfunction, more particularly as indicated in any embodiment above addressed to CASK protein dysfunction

In a particular embodiment, PPP2R1A protein dysfunction is as indicated above in the definition of protein dysfunction, more particularly as indicated in any embodiment above addressed to PPP2R1A protein dysfunction.

In a particular embodiment, step (i) of the methods of the invention comprises determining the expression level of at least miR-122-5p. In another particular embodiment, step (i) of the methods of the invention comprises determining the expression level of at least miR-2110.

In another particular embodiment, step (i) of the methods of the invention comprises determining the expression level of a miRNA or combination of miRNAs selected from:
- miR-122-5p and miR-140-5p, or
- miR_2110 and miR-217-5p.

In a more particular embodiment, step (i) of the methods of the invention comprises determining the expression level of a miRNA or combination of miRNAs selected from:
- miR-217-5p, miR-2110, miR-140-5p or
- miR-122-5p, miR-217-5p and miR-140-5p,
particularly comprises determining the expression level of the combination of miRNAs miR-217-5p, miR-2110, miR-140-5p.

In a particular embodiment, step (i) of the methods of the invention comprises determining the expression level of a miRNA or combination of miRNAs selected from those listed in table 1 below, particularly from those showing an AUC of at least 0.7 in table 1. In another embodiment, step (i) of the methods of the invention comprises determining the expression level of a miRNA or combination of miRNAs selected from those listed in table 1 below and showing an AUC of at least 0.72, more particularly of at least 0.7 3, in table 1. In another embodiment, step (i) of the methods of the invention comprises determining the expression level of a miRNA or combination of miRNAs selected from those listed in table 1 below and showing an AUC of at least 0.75 in table 1. In another embodiment, step (i) of the methods of the invention comprises determining the expression level of a miRNA or combination of miRNAs selected from those listed in table 1 below and showing an AUC of at least 0.77 in table 1. In another embodiment, step (i) of the methods of the invention comprises determining the expression level of a miRNA or combination of miRNAs selected from those listed in table 1 below and showing an AUC of at least 0.8 in table 1. In another embodiment, step (i) of the methods of the invention comprises determining the expression level of a miRNA or combination of miRNAs selected from those listed in table 1 below and showing an AUC of at least 0.81 in table 1, more particularly of at least 0.82 in table 1. In another embodiment, step (i) of the methods of the invention comprises determining the expression level of a miRNA or combination of miRNAs selected from those listed in table 1 below and showing an AUC of at least 0.83 in table 1, more particularly of at least 0.84 in table 1. In another embodiment, step (i) of the methods of the invention comprises determining the expression level of a miRNA or combination of miRNAs selected from those listed in table 1 below and showing an AUC of at least 0.85 in table 1. In another embodiment, step (i) of the methods of the invention comprises determining the expression level of a miRNA or combination of miRNAs selected from those listed in table 1 below and showing an AUC of at least 0.855 in table 1. In another embodiment, step (i) of the methods of the invention comprises determining the expression level of a miRNA or combination of miRNAs selected from those listed in table 1 below and showing an AUC of at least 0.858 in table 1. In another embodiment, step (i) of the methods of the invention comprises determining the expression level of a miRNA or combination of miRNAs selected from those listed in table 1 below and showing an AUC of at least 0.87 in table 1. In another embodiment, step (i) of the methods of the invention comprises determining the expression level of the combination of miRNAs miR-122-5p, miR-217-5p and miR-140-5p. In another embodiment, step (i) of the methods of the invention comprises determining the expression level of the combination of miRNAs miR-122-5p, miR-217-5p and miR-140-53p. In another embodiment, step (i) of the methods of the invention comprises determining the expression level of the combination of miRNAs miR-122-5p, miR-217-5p, miR-140-5, miR-2110 and miR-140-3p. In another embodiment, step (i) of the methods of the invention comprises determining the expression level of the combination of miRNAs miR-122-5p, miR-217-5p, miR-140-5 and miR-140-3p. In another embodiment, step (i) of the methods of the invention comprises determining the expression level of the combination of miRNAs miR-122-5p, and miR-140-5. In another embodiment, step (i) of the methods of the invention comprises determining the expression level of the combination of miRNAs miR-122-5p, miR-217-5p, miR-2110 and miR-140-3p. In another embodiment, step (i) of the methods of the invention comprises determining the expression level of the combination of miRNAs miR-122-5p, miR-217-5p, miR-2110 and miR-140-5p. In another embodiment, step (i) of the methods of the invention comprises determining the expression level of the combination of miRNAs miR-122-5p, miR-140-5, miR-2110 and miR-140-3p. In another embodiment, step (i) of the methods of the invention comprises determining the expression level of the combination of miRNAs miR-122-5p and miR-217-5p. In another embodiment, step (i) of the methods of the invention comprises determining the expression level of the combination of miRNAs miR-122-5p, miR-140-5, and miR-140-3p. In another embodiment, step (i) of the methods of the invention comprises determining the expression level of the combination of miRNAs miR-122-5p, miR-217-5p, miR-140-5, miR-2110 and miR-140-3p. In another embodiment, step (i) of the methods of the invention comprises determining the expression level of the combination of miRNAs miR-122-5p and miR-140-3p.

In a particular embodiment, the CA referred in the methods of the invention that comprise step (i) as defined in the previous paragraph, is a CA caused by or associated with PMM2-CDG.

In a particularly preferred embodiment, CA referred in any of the embodiments of the methods of the invention, is a CA caused or associated with PMM2-CDG, and step (i) comprises determining the expression level of miR-122-5p, miR-217-5p and miR-140-5p.

In a particular embodiment, step (i) of the methods of the invention comprises determining the expression level of a miRNA or combination of miRNAs selected from those listed in table 2 below, particularly from those showing an AUC of at least 0.6 in table 2. In another embodiment, step (i) of the methods of the invention comprises determining the expression level of a miRNA or combination of miRNAs selected from those listed in table 2 below and showing an AUC of at least 0.62, more particularly of at least 0.63, in table 2. In another embodiment, step (i) of the methods of the invention comprises determining the expression level of a miRNA or combination of miRNAs selected from those listed in table 2 below and showing an AUC of at least 0.64, more particularly of at least 0.65, in table 2. In another embodiment, step (i) of the methods of the invention comprises determining the expression level of a miRNA or combination of miRNAs selected from those listed in table 2 below and showing an AUC of at least 0.66, more particularly of at least 0.67, in table 2. In another embodiment, step (i) of the methods of the invention comprises determining the expression level of a miRNA or combination of miRNAs selected from those listed in table 2 below and showing an AUC of at least 0.68, more particularly of at least 0.69, in table 2. In another embodiment, step (i) of the methods of the invention comprises determining the expression level of a miRNA or combination of miRNAs selected from those listed in table 2 below and showing an AUC of at least 0.7, more particularly of at least 0.69, in table 2.

In a particular embodiment, the CA referred in the methods of the invention that comprise step (i) as defined in the previous paragraph, is a CA not caused by or associated to PMM2-CDG. In another particular embodiment, the CA referred in the methods of the invention that comprise step (i) as defined in the previous paragraph, is a CA caused by or associated with cerebellar hemorrhage in a premature infant, CACNA1G protein dysfunction, CACNA1A protein dysfunction, CASK protein dysfunction, PPP2R1A protein dysfunction or combinations thereof, particularly wherein the CA is not caused by or associated with PMM2-CDG.

In a particularly preferred embodiment, CA referred in any of the embodiments of the methods of the invention, is a CA not caused or associated with PMM2-CDG, and step (i) comprises determining the expression level of miR-2110, miR-217-5p, and miR-140-5p.

In a particularly preferred embodiment, CA referred in any of the embodiments of the invention is a CA not caused by, nor associated with PMM2_CDG, particularly caused by or associated with, more particularly caused by, CA caused by or associated with cerebellar hemorrhage in a premature infant, CACNA1G protein dysfunction, CACNA1A protein dysfunction, CASK protein dysfunction, PPP2R1A protein dysfunction or combinations thereof, particularly wherein the CA is not caused by or associated with PMM2-CDG, and step (i) of the methods of the invention comprises determining the expression level of a miRNA or combination of miRNAs, that shows an AUC in table 1 of less than 0.75, particularly less than 0.72, more particularly of less than 0.7, yet more particularly of less than 0.65. In a particular embodiment, the reference value used in step (iii) of the methods of the invention corresponds to the threshold value provided in table 1 below for the miRNA or combination of miRNAs whose expression level is determined in step (i) of the method, particularly when the expression level(s) determined in step (i) is/are provided in CPM. In another particular embodiment, the reference value used in step (iii) of the methods of the invention corresponds to +/- 2%, particularly +/- 1%, the threshold value provided in table 1 below for the miRNA or combination of miRNAs whose expression level is determined in step (i) of the method, particularly when the expression level(s) determined in step (i) is/are provided in CPM. In another particular embodiment, the reference value used in step (iii) of the methods of the invention corresponds to +/- 10% , particularly +/- 5%, the threshold value provided in table 1 below for the miRNA or combination of miRNAs whose expression level is determined in step (i) of the method, particularly when the expression level(s) determined in step (i) is/are provided in CPM. In another particular embodiment, the reference value used in step (iii) of the methods of the invention corresponds to +/- 20%, particularly +/- 15% the threshold value provided in table 1 below for the miRNA or combination of miRNAs whose expression level is determined in step (i) of the method, particularly when the expression level(s) determined in step (i) is/are provided in CPM.

In another particular embodiment, the reference value used in step (iii) of the methods of the invention is provided as a range of values, which corresponds to the range of threshold values indicated in table 1 below for the miRNA or combination of miRNAs whose expression level is determined in step (i) of the method, particularly when the expression level(s) determined in step (i) is/are provided in CPM. In another particular embodiment, the reference value determined in step (iii) of the invention is provided as a range of values, wherein the lower and upper values of the range correspond to +/- 2%, particularly +/-1% the lower and upper values, respectively, of the range of threshold values provided in table 1 below for the miRNA or combination of miRNAs whose expression level is determined in step (i) of the method, particularly when the expression level(s) determined in step (i) is/are provided in CPM. In another particular embodiment, the reference value used in step (iii) of the invention is provided as a range of values, the lower and upper values of the range correspond to +/- 5%, more particularly of +/- 10% the lower and upper values, respectively, of the range of threshold values provided in table 1 below for the miRNA or combination of miRNAs whose expression level is determined in step (i) of the method, particularly when the expression level(s) determined in step (i) is/are provided in CPM.

In a particularly preferred embodiment, the embodiments provided in the two paragraphs above apply to the methods of the invention wherein CA is a CA caused by or associated with, particularly caused by, PMM2-CDG. In a particular embodiment, the embodiments provided in the two paragraphs above apply to the methods of the invention wherein CA is a CA not caused by or associated with PMM2-CDG.

In a particular embodiment, the reference value used in step (iii) of the methods of the invention corresponds to the threshold value provided in table 2 below for the miRNA or combination of miRNAs whose expression level is determined in step (i) of the method, particularly when the expression level(s) determined in step (i) is/are provided in CPM. In another particular embodiment, the reference value used in step (iii) of the methods of the invention corresponds to +/- 2%, particularly +/- 1%, the threshold value provided in table 2 below for the miRNA or combination of miRNAs whose expression level is determined in step (i) of the method, particularly when the expression level(s) determined in step (i) is/are provided in CPM. In another particular embodiment, the reference value used in step (iii) of the methods of the invention corresponds to +/- 10%, particularly +/- 5%, the threshold value provided in table 2 below for the miRNA or combination of miRNAs whose expression level is determined in step (i) of the method, particularly when the expression level(s) determined in step (i) is/are provided in CPM. In another particular embodiment, the reference value used in step (iii) of the methods of the invention corresponds to +/- 20%, particularly +/- 15% the threshold value provided in table 2 below for the miRNA or combination of miRNAs whose expression level is determined in step (i) of the method, particularly when the expression level(s) determined in step (i) is/are provided in CPM.

In another particular embodiment, the reference value used in step (iii) of the methods of the invention is provided as a range of values, which corresponds to the range of threshold values indicated in table 2 below for the miRNA or combination of miRNAs whose expression level is determined in step (i) of the method, particularly when the expression level(s) determined in step (i) is/are provided in CPM. In another particular embodiment, the reference value determined in step (iii) of the invention is provided as a range of values, wherein the lower and upper values of the range correspond to +/- 2%, particularly +/-1%, the lower and upper values, respectively, of the range of threshold values provided in table 2 below for the miRNA or combination of miRNAs whose expression level is determined in step (i) of the method, particularly when the expression level(s) determined in step (i) is/are provided in CPM. In another particular embodiment, the reference value used in step (iii) of the invention is provided as a range of values, the lower and upper values of the range correspond to +/-5%, more particularly of +/- 10% the lower and upper values, respectively, of the range of threshold values provided in table 2 below for the miRNA or combination of miRNAs whose expression level is determined in step (i) of the method, particularly when the expression level(s) determined in step (i) is/are provided in CPM.

In a particularly preferred embodiment, the embodiments provided in the two paragraphs above apply to the methods of the invention wherein CA is a CA not caused by or associated with PMM2-CDG, more particularly is CA caused by or associated with, particularly caused by, cerebellar hemorrhage in a premature infant, CACNA1G protein dysfunction, CACNA1A protein dysfunction, CASK protein dysfunction, PPP2R1A protein dysfunction or combinations thereof. In a particular embodiment, the embodiments provided in the two paragraphs above apply to the methods of the invention wherein CA is a CA caused by or associated with PMM2-CDG.

In an embodiment, step (i) of the methods of the invention comprises, before determining the expression level of the corresponding miRNA or combination of miRNAs, isolating the RNA comprised in the isolated sample, particularly the C-RNA in the sample, more particularly wherein the sample is a blood sample, yet more particularly a plasma sample. Methods to isolate the RNA from a sample are well-known by an expert in the field and include phenol-chloroform, or guanidium-thiocyanate based methods, and particularly in case of C-RNA, miRNeasy Serum/Plasma Kit and following the manufacturer's instruction such as those from Qiagen ^{®}.

In another embodiment, step (i) of the method comprises, before determining the expression level of the corresponding miRNA or combination of miRNAs obtaining the cDNA corresponding the RNAs comprised in the sample, i.e. reverse-transcription of the RNA comprised in the isolated sample, particularly the C-RNA comprised in the sample. Methods for that purpose are well-known by an expert in the field and include **RT-**PCR or using a library preparation kit such as Qiagen^{®} QlAseq miRNA Library Kit and following the manufacturer's instructions.

In a further embodiment, step (i) of the method comprises, before determining the expression level of the corresponding miRNA or combination of miRNAs, sequencing the cDNAs corresponding to the RNAs comprised in the sample, particularly the C-RNAs comprised in the sample. Methods for that purpose are well-known by an expert in the field and include the use of NGS sequencers, such as Illumina^{®} NovaSeq sequencer and following the manufacturer's instructions.

In another embodiment, step (i) of the methods comprises, before determining the expression level of the corresponding miRNA or combination of miRNAs, bioinformatic analysis of the data obtained from sequencing the cDNAs corresponding to the RNAs, particularly C-RNAs, comprised in the sample, to determine the expression level of the miRNAs of interest. Bioinformatic tools for that purpose are well-known by an expert in the field and include those disclosed in the examples below.

In a particular embodiment, step (i) of the methods of the invention comprises:
(i.a) isolating the RNA comprised in the sample, particularly the C-RNA in the sample, more particularly wherein the sample is a blood sample, yet more particularly a plasma sample,
(i.b) synthesizing the cDNA corresponding the RNAs, or C-RNAs, obtained from step (i.a),
(i.c) sequencing the cDNAs, or cDNA libraries, obtained from step (i.b),
(i.d) analyzing with bioinformatic tools the sequencing data obtained from step (i.c) to determine the expression level in the sample of at least one miRNA indicated in step (i) of the methods of the invention.

In a particular embodiment, in the context of the present invention, the term "combinations thereof' refers to combinations of 2, 3, 4, or 5 elements. In a particular embodiment, the term "combinations thereof" refers to a combination of 2 elements. In another embodiment, the term "combinations thereof' refers to a combination of 3 elements. In another embodiment, the term "combinations thereof' refers to a combination of 4 elements. In another embodiment, the term "combinations thereof' refers to a combination of 5 elements.

In another particular embodiment of the present invention, the term "at least one" refers to 2, 3, 4, or 5 elements. In another particular embodiment, the term "at least one" refers to 1 element. In another particular embodiment, the term "at least one" refers to 2 elements. In another particular embodiment, the term "at least one" refers to 3 elements. In another particular embodiment, the term "at least one" refers to 4 elements. In another particular embodiment, the term "at least one" refers to 5 elements.

### B. In vitro method for deciding or recommending whether to initiate a therapeutic intervention in an individual suspicious of having cerebellar atrophy

In a second aspect, the invention is addressed to an *in vitro* method for deciding or recommending whether to initiate a therapeutic intervention in an individual suspicious of having cerebellar atrophy, wherein the method comprises steps (i)-(ii) as defined in the first aspect and (iii) if S calculated in step (ii) is equal or higher than the corresponding reference value, it is indicative that the individual has to start a therapeutic intervention.

In the present invention, "to initiate a therapeutic intervention" and the term "treat" are interchangeable. Thus, both are used herein to mean to intervene, or administer the subject with medicaments, to try to relieve, reduce or alleviate at least one symptom of a disease in the subject. For example, in relation to CA disease, these terms include intervening the subject (mainly through early stimulation programs, rehabilitation, physiotherapy or occupational therapy) or administering symptomatic medications to the subject (such as acetazolamide, verapamil, acetyl-leucine, antiepilleptics etc).

Accordingly, these terms also encompass trying to delay or prevent onset prior to clinical manifestation of a disease or symptom of a disease and/or reducing the risk of developing or worsening of a symptom of a disease. In a particular embodiment, the therapeutic intervention or treatment, as used herein, is for treatment or for slowing down the progression of CA. In a particular embodiment, the therapeutic intervention or treatment, as used herein, is for reducing at least one symptom associated with CA.

In a particular embodiment, when CA is caused by or associated with PMM2-CDG, the therapeutic intervention is selected from the list consisting of early stimulation programs, rehabilitation, physiotherapy, occupational therapy, administration of acetazolamide, administration of verapamil, administration of acetyl-leucine, administration of antiepileptics, and combinations thereof. In another particular embodiment, when CA is not caused or associated with PMM2-CDG, the therapeutic intervention is selected from the list consisting of early stimulation programs, rehabilitation, physiotherapy, occupational therapy, administration of acetazolamide, administration of verapamil, administration of antiepileptics, and combinations thereof, particularly, is selected from the list consisting of early stimulation programs, rehabilitation, physiotherapy, occupational therapy and combinations thereof. In another particular embodiment, when CA is caused or associated with CACNA1G protein dysfunction, the therapeutic intervention is selected from the list consisting of early stimulation programs, rehabilitation, physiotherapy, occupational and combinations thereof. In another particular embodiment, when CA is caused or associated with CACNA1A protein dysfunction, the therapeutic intervention is selected from the list consisting of early stimulation programs, rehabilitation, physiotherapy, occupational therapy, administration of acetazolamide, administration of verapamil, administration of antiepileptics, and combinations thereof. In another particular embodiment, when CA is caused or associated with CASK protein dysfunction, the therapeutic intervention is selected from the list consisting of early stimulation programs, rehabilitation, physiotherapy, occupational and combinations thereof. In another particular embodiment, when CA is caused or associated with PPP2R1A protein dysfunction, the therapeutic intervention is selected from the list consisting of early stimulation programs, rehabilitation, physiotherapy, occupational and combinations thereof.

The term "subject suspicious of suffering CA", as used herein, is a subject that has shown signs that could be assigned to CA or with high risk of having inherited the disease. In a particular embodiment, the subject suspicious of suffering CA referred to in the method of the second aspect is as the subject indicated in the definition of "subject" above with signs of being suffering CA, particularly, showing any of the symptoms of CA indicated in the definition of CA. In another particular embodiment, the subject suspicious of suffering CA referred to in the method of the second aspect is a subject with high risk of having inherited the disease, for instance, with at least one family member diagnosed with CA, or with a disease that causes or that is associated with CA. More particularly, the subject suspicious of suffering CA has at least one family member of first or second degree of consanguinity, particularly of first degree of consanguinity, diagnosed with CA or with a disease that causes or that is associated with CA. In another particular embodiment, the subject suspicious of suffering CA referred to in the method of the second aspect is a subject with at least one family member of first or second degree of consanguinity, particularly of first degree of consanguinity, diagnosed with CA or with a disease that causes or that is associated with CA, and that in addition, shows signs of being suffering CA as for instance the symptoms of CA indicated in the definition of CA. In a particular embodiment, a disease that causes or that is associated with CA, as used herein, refers to PMM2-CDG, CACNA1G protein dysfunction, CACNA1A protein dysfunction, CASK protein dysfunction, and/or PPP2R1A protein dysfunction, particularly CACNA1A protein dysfunction, more particularly PMM2-CDG.

As well understood by a skilled person, the reference value used in step (iii) of the method of the second aspect of the invention, is as the reference value used in step (iii) of the method of the first aspect of the invention. **The** definition and embodiments provided in the first aspect for defining or determining the "reference value" are equally applicable to said term in the second aspect of the invention.

**The** rest of terms in the context of the present aspect of the invention have the meaning defined for the first aspect of the invention. All the embodiments and definitions provided in the first aspect of the invention, particularly to define or specify step (i) of the method of the first aspect, are equally applicable to the method of the second aspect of the invention. All the embodiments and definitions provided in the first aspect of the invention to define or specify step (ii) of the method of the first aspect are equally applicable to the method of the second aspect of the invention.

### C. In vitro method for determining the efficacy of a therapeutic intervention in a patient already diagnosed with CA.

In a third aspect, the invention is addressed to an *in vitro* method for determining the efficacy of a therapeutic intervention in a patient already diagnosed with CA, wherein the method comprises performing steps (i) and (ii) as defined in the first aspect of the invention before and after starting the therapeutic intervention, and if the level of S calculated in step (ii) after starting the therapeutic intervention is lower than the level of S calculated in step (ii) before starting the therapeutic intervention, it is indicative that the therapeutic intervention is effective in the treatment of CA.

The expression "efficacy of a therapeutic intervention", or "therapeutic intervention is effective in the treatment of CA", as used herein, refers to the fact that a patient already diagnosed with CA and that has received a therapeutic intervention for the treatment of CA reacts favorably to the therapeutic intervention. As well understood by a skilled person, a therapy effective in the treatment of CA can relieve, reduce or alleviate at least one symptom of the disease in the individual. Accordingly, these terms also encompass delaying or preventing onset prior to clinical manifestation of the disease or symptom of the disease and/or reducing the risk of developing or worsening of a symptom of the disease. Known therapeutic interventions for that purpose have already been provided in the second aspect of the invention and can be applied to the third aspect of the invention.

In a particular embodiment, the therapeutic intervention or treatment, as used herein, is for the treatment of CA, particularly as defined in the second aspect of the invention. In a particular embodiment, the type of CA is as in the method of the first or second aspect, and the treatment is as indicated in the second aspect for said type of CA.

The term "the level of S calculated in step (ii) after starting the therapeutic intervention is lower than the level of S calculated in step (ii) before starting the therapeutic intervention" as used herein, refers to the fact that the level of S calculated in step (ii) after starting the therapeutic intervention is at least about 1%, 2%, 3%, 4%, 5%, 6%, 7%, 8%, 9%, 10%, 12%, 15%, 20%, 25%, 30%, 40%, 50%, 75%, 90%, 95%, 100%, 125%, 150%, 175%, 200% lower than the level of S calculated in step (ii) before starting the therapeutic intervention.

The term "after starting the therapeutic intervention" refers to a time point after the administration of the therapeutic intervention to the patient, particularly, to a time point immediately after the administration of the therapeutic intervention to the patient. In another particular embodiment, the term refers to at least 5 minutes, at least 10 minutes, at least 15 minutes, at least 30 minutes, at least 1 hour, at least 2 hours, at least 3 hours, at least 4 hours, at least 5 hours, at least 6 hours, at least 8 hours, at least 10 hours, at least 12 hours, at least 18 hours, at least 24h, at least 36 hours, at least 2 days, at least 3 days, at least 4 days, at least 5 days, at least 1 week, at least 2 weeks, at least 3 weeks, at least 4 weeks, at least 2 months, at least 3 months, at least 4 months, at least 5 months, at least 6 months, at least 12 months, at least 18 months, at least 24 months, after the last administration of the therapeutic intervention to the subject.

The term "before starting the therapeutic intervention", as used herein, refers to any time before the start of the therapeutic intervention. In a particular embodiment, said term refers to a time point immediately before starting the therapeutic intervention. In another particular embodiment, said term refers to at least 5 minutes, at least 10 minutes, at least 15 minutes, at least 30 minutes, at least 1 hour, at least 2 hours, at least 3 hours, at least 4 hours, at least 5 hours, at least 6 hours, at least 8 hours, at least 10 hours, at least 12 hours, at least 18 hours, at least 24h, at least 36 hours, at least 2 days, at least 3 days, at least 4 days, at least 5 days, at least 1 week, at least 2 weeks, at least 3 weeks, at least 4 weeks, at least 5 weeks, at least 1 month, at least 2 months, at least 3 months, at least 6 months, at least 9 months, at least 1 year, at least 1.5 years, at least 2 years, at least 3 years, before starting the therapeutic intervention. In a particular embodiment, "before starting the therapeutic intervention", refers to any time between the time when the subject was diagnosed with CA and the start of the therapeutic intervention. In another particular embodiment, the term refers to at least 5 minutes, at least 10 minutes, at least 15 minutes, at least 30 minutes, at least 1 hour, at least 2 hours, at least 3 hours, at least 4 hours, at least 5 hours, at least 6 hours, at least 8 hours, at least 10 hours, at least 12 hours, at least 18 hours, at least 24h, at least 36 hours, at least 2 days, at least 3 days, at least 4 days, at least 5 days, at least 1 week, at least 2 weeks, at least 3 weeks, at least 4 weeks, at least 5 weeks, at least 1 month, at least 2 months, at least 3 months, at least 6 months, at least 1 year, at least 2 years, after being diagnosed with CA, provided it is before starting the therapeutic intervention of study.

In a particular embodiment, the subject may have received other therapeutic interventions for the treatment of CA, previous to the one being studied in the third aspect of the invention. Thus, in a particular embodiment, the subject of study in the third aspect of the invention has already received a therapeutic intervention for the treatment of CA, particularly for the treatment of PMM2-CDG, prior to performing the method, different from that being studied in the method of the third aspect. In another particular embodiment, the therapeutic intervention of the third aspect of the invention is a second line or third line therapeutic treatment for the treatment of CA applied to the subject.

The rest of terms in the context of the present aspect of the invention have the meaning defined for the first and second aspects of the invention. All the embodiments and definitions provided in the first and second aspects of the invention are equally applicable to the method of the third aspect of the invention. In a particular embodiment, the definitions and embodiments of step (i) of the method of the first and second aspects of the invention, are equally applicable to the method of the third aspect. In another embodiment, the definitions and embodiments of step (ii) of the method of the first and second aspects of the invention, are equally applicable to the method of the third aspect.

### D. A method for classifying a subject into a subject cohort.

In a fourth aspect, the invention is addressed to a method for classifying a subject into a subject cohort, wherein the method comprises steps (i)-(ii) as defined in the first aspect and (iii) classifying the subject into:
- a cohort of subjects with high probability of suffering cerebellar atrophy if S calculated in step (ii) is equal or higher than the corresponding reference value, or
- a cohort of subjects with low probability of suffering cerebellar atrophy if S calculated in step (ii) is lower than the corresponding reference value.

The term "cohort" or "patient cohort" as used herein, refers to a group of subjects affected by common diseases, environmental or temporal influences, treatments, or other traits whose progress is assessed in a research or clinical study. In the present invention the trait common for all the subjects within a cohort is that they have or do not have CA.

The term "high probability", as used herein in the method for classifying a subject into a subject cohort, relates to the situation where the subject is considered to have CA, although not necessarily 100% sure. In a particular embodiment, the term "high probability" as used herein refers to the situation where the subject is preliminarily considered to have CA. In another particular embodiment, the term "high probability" in the context of the method for determining the probability of a subject to have CA, relates to the situation where the subject shows at least 5 %, at least 10%, at least 20%, at least 30%, at least 40%, at least 50%, at least 60%, at least 70%, at least 80%, at least 90%, or 100% probabilities to have CA.

The term "low probability", as used herein in the method for classifying a subject into a subject cohort, relates to the situation where the subject is considered to not have CA, although not necessarily 100% sure. In a particular embodiment, the term "low probability" as used herein refers to the situation where the subject is preliminarily considered to not have CA. In another particular embodiment, the term "low probability" in the context of the method for determining the probability of a subject to have CA, relates to the situation where the subject shows less than 70%, less than 60%, less than 50%, less than 45%, less than 40%, less than 30%, less than 25%, less than 20%, less than 15%, less than 10%, less than 7%, less than 5%, less than 3%, less than 2%, less than 1%, less than 0.5%, less than 0.01% of probabilities to have CA.

In a particular embodiment, the subjects with low probability of suffering CA are considered to be healthy subjects.

In a particular embodiment, the method of the fourth aspect comprises a step (iv), wherein it is recommended or determined that the subjects classified in a cohort of subjects having a high probability to have CA, perform an additional CA diagnostic method. More particularly, the CA diagnostic method is any of those known by an expert in the field, in particular comprises or consists of performing a transfontanellar ultrasonography (in infants), MRI or a CT scan.

In a particular embodiment, cerebellar atrophy referred to in any of the embodiments of the fourth aspect is as indicated in any of the embodiments of the first aspect of the invention. In a more particular embodiment, cerebellar atrophy referred in step (iii) or (iv) of the fourth aspect is as indicated in any of the embodiments of the first aspect of the invention. In another particular embodiment, the type of cerebellar atrophy referred to in any of the embodiments of the fourth aspect is the same as in the first aspect of the invention. In a more particular embodiment, the type of cerebellar atrophy referred to in step (iii) and (iv) of the fourth aspect is the same as in the first aspect of the invention. In a particular embodiment, cerebellar atrophy referred to in any of the embodiments of the fourth aspect is as indicated in any of the embodiments of the first aspect of the invention. In a more particular embodiment, cerebellar atrophy referred to in step (iii) and (iv) of the fourth aspect is as indicated in any of the embodiments of the first aspect of the invention. In another particular embodiment, the type of cerebellar atrophy referred to in any of the embodiments of the fourth aspect is the same as in the first aspect of the invention. In a more particular embodiment, the type of cerebellar atrophy referred to in step (iii) and (iv) of the fourth aspect is the same as in the first aspect of the invention.

In another embodiment, the miRNA or combination of miRNAs whose expression level is determined in step (i) of the method of the fourth aspect of the invention is the miRNA or combination of miRNAs as indicated in any embodiment of the first aspect of the invention, and the CA referred in step (iii) of the fourth aspect is as the CA indicated in the first aspect for said miRNA or combination of miRNAs. In another embodiment, the miRNA or combination of miRNAs whose expression level is determined in step (i) of the method of the fourth aspect of the invention is the miRNA or combination of miRNAs as indicated in any embodiment of the first aspect of the invention, and the CA referred in step (iv) of the fourth aspect is as the CA indicated in the first aspect for said miRNA or combination of miRNAs. In another embodiment, the miRNA or combination of miRNAs whose expression level is determined in step (i) of the method of the fourth aspect of the invention is the miRNA or combination of miRNAs as indicated in any embodiment of the first aspect of the invention, and the CA referred in step (iv) of the fourth aspect is as the CA indicated in the first aspect for said miRNA or combination of miRNAs. In another embodiment, the miRNA or combination of miRNAs whose expression level is determined in step (i) of the method of the fourth aspect of the invention is the miRNA or combination of miRNAs as indicated in any embodiment of the first aspect of the invention, and the CA referred in step (iv) of the fourth aspect is as the CA indicated in the first aspect for said miRNA or combination of miRNAs.

As well understood by a skilled person, the reference value used in step (iii) of the method of the fourth aspect of the invention, is as the reference value used in step (iii) of the method of the first aspect of the invention. The definition and embodiments provided in the first aspect for the definition or specification of "reference value" are equally applicable to said term in the fourth aspect of the invention.

The rest of terms in the context of the present aspect of the invention have the meaning defined in the first, or second aspects of the invention. All the embodiments and definitions provided in the first and second aspects of the invention, particularly to define or specify step (i) and step (ii) of the methods, are equally applicable to the method of the fourth aspect of the invention.

The *in vitro* methods of the invention provide diagnostic, stratification and/or monitoring information. In one embodiment, the methods of the invention further comprise the steps of (iv) (or (v) for the method of the fourth aspect comprising a step (iv)) collecting the diagnostic, stratification, and/or monitoring information, and (v) (or (vi) for the method of the fourth aspect comprising a step (iv)) saving the information in a data carrier.

In the sense of the invention a "data carrier" is to be understood as any means that contains meaningful information data for the diagnosis and/or prognosis of CA in an individual, such as paper. The carrier may also be any entity or device capable of carrying the prognosis data. For example, the carrier may comprise a storage medium, such as a ROM, for example a CD ROM or a semiconductor ROM, or a magnetic recording medium, for example a floppy disc or hard disk. Further, the carrier may be a transmissible carrier such as an electrical or optical signal, which may be conveyed via electrical or optical cable or by radio or other means. When the diagnosis/prognosis/monitoring data are embodied in a signal that may be conveyed directly by a cable or other device or means, the carrier may be constituted by such cable or other device or means. Other carriers relate to USB devices and computer archives. Examples of suitable data carriers are paper, CDs, USB, computer archives in PCs, or sound registration with the same information.

### II. Uses of the invention.

In a fifth aspect, the invention is addressed to the use of at least one miRNA selected from the group consisting of miR-122-5p, miR-2110, miR-217-5p, miR-140-5p, miR-140-3p, and combinations thereof, as a biomarker for the diagnosis of cerebellar atrophy; or as a biomarker for deciding or recommending whether to initiate a therapeutic intervention in an individual suspicious of having cerebellar atrophy; or as a biomarker for determining the efficacy of a therapeutic intervention in a patient already diagnosed with cerebellar atrophy; or as a biomarker for classifying a subject into a cohort of subjects with high probabilities of having cerebellar atrophy or of subjects with low probabilities of having cerebellar atrophy.

In a sixth aspect, the invention is addressed to the use of a kit for the diagnosis of cerebellar atrophy in an individual; or for deciding or recommending whether to initiate a therapeutic intervention in an individual suspicious of having cerebellar atrophy; or for determining the efficacy of a therapeutic intervention in a patient already diagnosed with cerebellar atrophy; or for classifying a subject into a subject cohort of subjects with high probabilities of having cerebellar atrophy or of subjects with low probabilities of having cerebellar atrophy; the kit comprising means for detecting the level of expression of at least one miRNA selected from the list consisting of miR-122-5p, miR-2110, miR-140-5p, miR-217-5p, miR-140-3p, and combinations thereof, and optionally a solid support.

In an embodiment of the fifth aspect, the miRNA or combination of miRNAs used in the method of the fifth or sixth aspect, corresponds to the miRNA or combination of miRNAs whose expression level is determined in step (i) of the method of the first aspect of the invention.

In an embodiment of the sixth aspect of the invention, the means for detecting the expression level of the at least one miRNA correspond to means for detecting the miRNA or combination of miRNAs whose expression level is determined in step (i) of the method of the first aspect of the invention. In another embodiment of the sixth aspect, the at least one miRNA selected from the list consisting of miR-122-5p, miR-2110, miR-140-5p, miR-217-5p, miR-140-3p, and combinations thereof, corresponds to the miRNA or combination of miRNAs whose expression level is determined in step (i) of the method of the first aspect of the invention.

In another particular embodiment of the sixth aspect of the invention, the expression level of the at least one miRNA or combination of miRNAs as defined herein corresponds to the level of RNA transcribed from the corresponding gene, particularly in a sample or volume of body fluid, as defined in the first aspect of the invention. In another particular embodiment of the sixth aspect, the expression level is provided in CPM.

In another particular embodiment, the kit is an RNA-seq, RT-PCR, RT-qPCR, PCR, or an RNA microarray kit. In another embodiment of the sixth aspect, the means for detecting the expression level of the miRNA or combination of miRNAs as defined herein are primer oligonucleotides. In a particular embodiment, the primer oligonucleotides specifically hybridize or are complementary to one or more nucleotides of the miRNA or combination of miRNAs as defined herein. In a particular embodiment, the primers of the kit are non-naturally occurring oligonucleotides. In a more particular embodiment, the primers of the kit comprise artificial (i.e. human-made) modifications in the sequence or structure of the primers, as compared to the nucleotide sequence in the human genome that is complementary to the primer's target sequence. Such modifications can include artificial nucleotides, or artificial nucleotide sequences, comprised within the binding sequence of the primer, or attached or directly attached to the binding sequence of the primer, particularly to the 5'- or 3'-end of the binding sequence of the primer. As well understood by a skilled person, the binding sequence of the primer is the sequence that is complementary to the target sequence of the primer, wherein said binding sequence does not need to be 100% complementary to its target sequence. The terms "artificial nucleotide" or "artificial nucleotide sequences" as used herein, refer to a nucleotide or nucleotide sequence, respectively, that are not comprised in the sequence of the human genome that is complementary to the target sequence of the primer. Non-limitative examples of artificial modifications can include labels or tags attached or directly attached to the primers, particularly, fluorescently labelled tags. More particularly, the primers are modified primers, such as LNA (locked-nucleic acid) or PNA (peptide nucleic acid) primers. In another particular embodiment, the primers comprise at least one nucleotide not found in the sequence of the human genome that is complementary, particularly 100% complementary, to the primer's target sequence as well.

In a particular embodiment, the kit of the sixth aspect of the invention comprises a solid support.

In the present invention, the term "solid support" includes a nitrocellulose membrane, glass or a polymer. The most commonly used polymers being cellulose, polyacrylamide, nylon, polystyrene, polyvinyl chloride or polypropylene. The solid supports may be in the form of strips, tubes, beads, discs or microplates, or any other surface suitable for conducting gene expression level analysis, particularly RNA expression level analysis.

In a particular embodiment of the sixth aspect of the invention, the means for detecting the level of expression of the selected miRNA(s), particularly the oligonucleotide primers, comprised in the kit comprise at least 10%, at least 20%, at least 30%, at least 40%, at least 50%, at least 60%, at least 70%, at least 80%, at least 90%, at least 95%, or 100% of the total amount of reagents forming the kits used in the invention, preferably of the total amount of reagents adequate for the determination of the level of expression of one or more miRNAs forming the kit.

In another embodiment of the sixth aspect, the kit comprises instructions on how to use it, nucleotides, a polymerization enzyme (e.g. Taq polymerase) and buffer solution(s), particularly, in addition to the primers as defined herein.

The invention also contemplates a kit comprising means as defined above for detecting the level of expression of a miRNA or combination of miRNAs as indicated in any of the embodiments of the sixth aspect. In a particular embodiment, said kit comprises a solid support or instructions to perform any of the methods as defined in the first to fourth aspects. In another particular embodiment, the kit defined herein comprises a solid support and instructions to perform any of the methods as defined in the first to fourth aspects. In a particular embodiment, the kit of the present aspect is as the kit defined in the sixth aspect of the invention.

In an embodiment, the use of the fifth aspect or the use of the sixth aspect of the invention is for performing any of the methods of the invention. Thus, in a particular embodiment, the use of the fifth or sixth aspect is for performing the method of the first, second, third or fourth aspect of the invention, particularly the method of the first aspect of the invention. In another particular embodiment, the use of the fifth or sixth aspect is for performing the method of the second aspect of the invention. In another particular embodiment, the use of the fifth or sixth aspect is for performing the method of the third aspect of the invention. In another particular embodiment, the use of the fifth or sixth aspect is for performing the method of the fourth aspect of the invention.

All the above embodiments, related to the methods of the invention, are also embodiments of the uses provided by the present invention, too. Thus, all the definitions and embodiments provided in the first, second, third and fourth aspects of the invention are equally applicable to the fifth aspect of the invention and to the sixth aspect of the invention.

In a further aspect, the invention provides a method for diagnosing and treating CA, said method comprising steps (i) and (ii) as defined in the first aspect of the invention, (iii) identifying the subject having CA when S calculated in step (ii) is equal or higher than a corresponding reference value, and (iv) administering a treatment to the patient diagnosed as having CA. In a particular embodiment, the treatment is for CA, particularly it is selected from those indicated in the second aspect of the invention.

In a further aspect, the invention provides a method for diagnosing and treating CA, said method comprising steps (i), (ii) and (iii) as defined in the method to recommend or determine to perform a method for the diagnosis of CA in a patient, (iv) performing a diagnostic method when recommended or determined in step (iii), (v) identifying the subject as having CA when the diagnostic method performed in step (iv) diagnoses the patient as suffering CA, and (vi) administering a treatment to the patient diagnosed as having CA. In a particular embodiment, the treatment is a treatment for CA, particularly, it is selected from the treatment of CA indicated in the second aspect of the invention. In another embodiment, the CA diagnostic method is any of those known by an expert in the field, in particular comprises or consists of performing an transfontanellar ultrasonography (in infants) MRI or a CT scan.

In a particular embodiment of the previous two aspects, the CA referred therein is selected from a CA caused or associated with PMM2-CDG, cerebellar hemorrhage in a premature infant, CACNA1G protein dysfunction, CACNA1A protein dysfunction, CASK protein dysfunction, PPP2R1A protein dysfunction and combinations thereof. In a more particular embodiment, the CA referred therein is caused or associated with PMM2-CDG. In a yet more particular embodiment, the CA referred therein is as indicated in any of the embodiments of the first aspect of the invention.

In another particular embodiment, the treatment for CA referred in the previous two aspects, is as indicated in the second aspect of the invention, particularly is a treatment as indicated in the second aspect for the particular CA referred in said previous two aspects.

Finally, in another aspect, the invention relates to an algorithm for carrying out any of the methods of the invention as defined in the above aspects. In the sense of the invention, the term "algorithm" is also synonymous with a panel or decision diagrams, predictors and combinations of data to correctly categorize an individual sample.

According to aspects and embodiments of the invention, the methods of the invention can be performed using a mathematical algorithm that assesses a detectable level of RNA corresponding to the gene or combination of genes as described herein in any of the methods of the invention, either in conjunction with or independent of other clinical parameters, to correctly categorize an individual sample as originating from a healthy patient, or from a patient having CA .

The classification algorithm may be as simple as determining whether or not the amount of a specific biomarker or subset of biomarkers measured are above or below the reference level as defined in the first aspect of the invention. When multiple biomarkers are used, the classification algorithm may be a linear regression formula. Alternatively, the classification algorithm may be the product of any of a number of learning algorithms. In the case of complex classification algorithms, it may be necessary to perform the algorithm on the data, thereby determining the classification, using a computer, e.g., a programmable digital computer. In either case, one can then record the status on tangible medium, for example, in computer-readable format such as a memory drive or disk or simply printed on paper. The result also could be reported on a computer screen. This algorithm is used as a CA diagnostic method or as a method as described in any of the aspects of the invention and is in particular part of the kits used in the sixth aspect of the invention, particularly for carrying out the methods disclosed in former aspects.

Throughout the description and numbered embodiments, the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention.

For completeness, the present description is also disclosed in the following numbered embodiments:
1- An *in vitro* method for the diagnosis of cerebellar atrophy in a subject comprising:
   (i) determining, in a sample isolated from the subject, the expression level of at least one miRNA selected from the group consisting of miR-122-5p, miR-2110, miR-140-5p, miR-217-5p, miR-140-3p, and combinations thereof,
   (ii) calculating a variable S, wherein: $S = A - B - C + D - E$ and wherein:
      - A is the expression level of miR-122-5p determined in step (i), or 0 when the expression level of miR-122-5p is not determined in step (i),
      - B is the expression level of miR-2110 determined in step (i), or 0 when the expression level of miR-2110 is not determined in step (i),
      - C is the expression level of miR-140-5p determined in step (i), or 0 when the expression level of miR-140-5p is not determined in step (i),
      - D is the expression level of miR-217-5p determined in step (i), or 0 when the expression level of miR-217-5p is not determined in step (i),
      - E is the expression level of miR-140-3p determined in step (i), or 0 when the expression level of miR-140-3p is not determined in step (i), and
   (iii) diagnosing the subject as having cerebellar atrophy if S calculated in step (ii) is equal or higher than a corresponding reference value and as not having cerebellar atrophy if S calculated in step (ii) is lower than the corresponding reference value.
2- The method according to numbered embodiment 1, wherein step (i) further comprises determining in the sample isolated from the subject, the expression level of at least one gene and/or additional miRNA and the variable S in step (ii) is: $S = A - B - C + D - E + {\sum }_{i = 1}^{N} {a}_{i} {F}_{i} ,$ wherein
   F is the expression level of the at least one gene or additional miRNA, N is the number of additional miRNAs whose expression level is determined in step (i) and a is (1) if an increased expression level of the corresponding gene or miRNA is associated to having cerebellar atrophy or is (-1) if a decreased expression level of the corresponding gene or miRNA is associated to having cerebellar atrophy.
3- The method according to numbered embodiments 1 or 2, wherein S= A - B - C + D - E + G, wherein:
   - A, B, C, D and E are as defined in numbered embodiment 1;
      and:
   - G is obtained applying formula $G = {\sum }_{i = 1}^{N} ai Fi$, when step (i) further comprises determining the
      expression level of at least one gene and/or additional miRNA, particularly of at least one gene or additional miRNA, more particularly when step (i) further comprises determining the expression level of at least one additional miRNA; wherein:
      - F, N and a are as defined in numbered embodiment 2;
         Or
      - G is 0 when step (i) does not further comprise determining the expression level of at least one gene and/or additional miRNA.
4- The method according to any one of numbered embodiments 2 or 3, wherein the at least one gene and/or additional miRNA consists of at least one additional miRNA.
5- The method according to any one of numbered embodiments 1-4, wherein step (i) comprises determining in the isolated sample the expression level of miR-122-5p.
6- The method according to any one of numbered embodiments 1-5, wherein step (i) comprises determining in the isolated sample the expression level of miR-2110.
7- The method according to any one of numbered embodiments 1-6, wherein step (i) comprises determining in the isolated sample the expression level of miR-140-5p.
8- The method according to any one of numbered embodiments 1-7, wherein step (i) comprises determining in the isolated sample the expression level of miR-140-3p.
9- The method according to any one of numbered embodiments 1-8, wherein step (i) comprises determining in the isolated sample the expression level of miR-217-5p.
10- The method according to any one of numbered embodiments 1-9, wherein step (i) comprises determining in the isolated sample the expression level of a miRNA or combinations of miRNA selected from the list disclosed in table 1.
11- The method according to numbered embodiment 10, wherein the miRNA or combination of miRNAs selected from the list disclosed in table 1 is a miRNA or combination of miRNAs shown in table 1 to provide an AUC equal or higher than 0.7.
12- The method according to numbered embodiment 10, the miRNA or combination of miRNAs selected from the list disclosed in table 1 is a miRNA or combination of miRNAs shown in table 1 to provide an AUC equal or higher than 0.75.
13- The method according to numbered embodiment 10, the miRNA or combination of miRNAs selected from the list disclosed in table 1 is a miRNA or combination of miRNAs shown in table 1 to provide an AUC equal or higher than 0.8.
14- The method according to numbered embodiment 10, the miRNA or combination of miRNAs selected from the list disclosed in table 1 is a miRNA or combination of miRNAs shown in table 1 to provide an AUC equal or higher than 0.81.
15- The method according to numbered embodiment 10, the miRNA or combination of miRNAs selected from the list disclosed in table 1 is a miRNA or combination of miRNAs shown in table 1 to provide an AUC equal or higher than 0.82.
16- The method according to numbered embodiment 10, the miRNA or combination of miRNAs selected from the list disclosed in table 1 is a miRNA or combination of miRNAs shown in table 1 to provide an AUC equal or higher than 0.83.
17- The method according to numbered embodiment 10, the miRNA or combination of miRNAs selected from the list disclosed in table 1 is a miRNA or combination of miRNAs shown in table 1 to provide an AUC equal or higher than 0.84.
18- The method according to numbered embodiment 10, the miRNA or combination of miRNAs selected from the list disclosed in table 1 is a miRNA or combination of miRNAs shown in table 1 to provide an AUC equal or higher than 0.85, particularly equal or higher than 0.855, more particularly, equal or higher than 0.858.
19- The method according to any one of numbered embodiments 1-18, wherein step (i) comprises determining in the isolated sample the expression level of miR-122-5p, miR-217-5p, miR-140-5p.
20- The method according to any one of numbered embodiments 1-9, wherein step (i) comprises determining in the isolated sample the expression level of a miRNA or combinations of miRNA selected from the list disclosed in table 2.
21- The method according to numbered embodiment 20, wherein the miRNA or combination of miRNAs selected from the list disclosed in table 2 is a miRNA or combination of miRNAs shown in table 2 to provide an AUC equal or higher than 0.6.
22- The method according to any one of numbered embodiments 20-21, wherein the miRNA or combination of miRNAs selected from the list disclosed in table 2 is a miRNA or combination of miRNAs shown in table 2 to provide an AUC equal or higher than 0.62.
23- The method according to any one of numbered embodiments 20-22, wherein the miRNA or combination of miRNAs selected from the list disclosed in table 2 is a miRNA or combination of miRNAs shown in table 2 to provide an AUC equal or higher than 0.65.
24- The method according to any one of numbered embodiments 20-23, wherein the miRNA or combination of miRNAs selected from the list disclosed in table 2 is a miRNA or combination of miRNAs shown in table 2 to provide an AUC equal or higher than 0.67, particularly, equal or higher than 0.68, more particularly, equal or higher than 0.688.
25- The method according to any one of numbered embodiments 20-24, wherein the miRNA or combination of miRNAs selected from the list disclosed in table 2 is a miRNA or combination of miRNAs shown in table 2 to provide an AUC equal or higher than 0.7.
26- The method according to numbered embodiment 10, wherein the miRNA or combination of miRNAs selected from the list disclosed in table 1 is a miRNA or combination of miRNAs shown in table 1 to provide an AUC equal or lower than 0.75, particularly lower than 0.7, more particularly lower than 0.65 and the CA is not caused by nor associated with Phosphomannomutase 2 Congenital Disorder of Glycosylation, more particularly is caused by or associated with a cerebellar hemorrhage in a premature infant, CACNA1G protein dysfunction, CACNA1A protein dysfunction, CASK protein dysfunction, PPP2R1A protein dysfunction or combinations thereof.
27- The method according to any one of numbered embodiments 1-9, 20-26, wherein step (i) comprises determining in the isolated sample the expression level of miR-2110, miR-217-5p, and miR-140-5p.
28- The method according to any one of numbered embodiments 1-27, wherein step (i) comprises determining in the isolated sample, the expression level of:
   - miR-122-5p, miR-217-5p and miR-140-5p
   - miR-122-5p,
   - miR-122-5p, miR-217-5p and miR-140-3p,
   - miR-122-5p, miR-217-5p, miR-140-5p and miR-140-3p
   - miR-2110 and miR-217-5p,
   - miR-2110 and miR-140-5p,
   - miR-2110, miR-140-5p and miR-217-5p, or
   - miR-122-5p, miR-217-5p, miR-140-5p, miR-2110 and miR-140-3p.
29- The method according to any one of numbered embodiments 1-9, wherein step (i) comprises determining in the isolated sample, the expression level of miR-122-5p, miR-217-5p, miR-140-5p, miR_2110 and miR-140-3p.
30- An *in vitro* method for deciding or recommending whether to initiate a therapeutic intervention in an individual suspicious of having cerebellar atrophy, wherein the method comprises steps (i)-(ii) as defined in any one of numbered embodiments 1-29, and (iii) if S calculated in step (ii) is equal or higher than the corresponding reference value, it is indicative that the individual has to start a therapeutic intervention.
31- An *in vitro* method for determining the efficacy of a therapeutic intervention in a patient already diagnosed with cerebellar atrophy, wherein the method comprises performing steps (i)-(ii) as defined in any one of numbered embodiments 1-29 before and after starting the therapeutic intervention, and if S calculated in step (ii) obtained after starting the therapeutic intervention, is lower than S calculated in step (ii) obtained before starting the therapeutic intervention, it is indicative that the therapeutic intervention is effective in the treatment of cerebellar atrophy.
32- A method for classifying a subject into a subject cohort, wherein the method comprises steps (i)-(ii) as defined in any one of numbered embodiments 1-29 and (iii) classifying the subject into:
   - a cohort of subjects with high probability of suffering cerebellar atrophy if S calculated in step (ii) is equal or higher than the corresponding reference value, or
   - a cohort of subjects with low probability of suffering cerebellar atrophy if S calculated in step (ii) is lower than the corresponding reference value.
33- The method according to numbered embodiment 32, wherein it is decided or recommended to perform a method for the diagnosis of cerebellar atrophy to the subject with high probability of having cerebellar atrophy.
34- The method according to any one of numbered embodiments 32-33, wherein it is decided or recommended to not perform a method for the diagnosis of cerebellar atrophy to the subject with low probability of having cerebellar atrophy.
35- An in vitro screening method for the identification of subjects to whom a diagnostic method of cerebellar atrophy is to be performed, wherein the method comprises steps (i) and (ii) as defined in any one of numbered embodiments 1-29 and (iii) identifying the subject as a subject to whom a diagnostic method of CA is to be performed, if S calculated in step (ii) is equal or higher than the corresponding reference value, or as a subject to whom a diagnostic method of cerebellar atrophy is not to be performed if S calculated in step (ii) is lower than the corresponding reference value.
36- The method according to any one numbered embodiments 32-35, wherein the method for the diagnosis of cerebellar atrophy is different from the diagnostic method as defined in any one of numbered embodiments 1-29, particularly comprises or consists of performing transfontanellar ultrasonography, magnetic resonance imaging or a computed tomography scan, more particularly, magnetic resonance imaging or a computed tomography scan.
37- The method according to any one of the previous numbered embodiments wherein the sample is a blood sample, in particular, a plasma sample.
38- The method according to any one of the previous numbered embodiments, wherein the expression level determined in step (i) is provided in counts per million (CPM).
39- The method according to any one of the previous numbered embodiments wherein determining the expression level in step (i) is performed by RNA sequencing.
40- The method according to any one numbered embodiments 1-39, wherein the reference value used in step (iii) corresponds to the threshold value provided in table 1 for the miRNA or combination of miRNAs whose expression level is determined in step (i), particularly when the expression level(s) determined in step (i) is/are provided in counts per million.
41- The method according to any one of numbered embodiments 1- 39, wherein the reference value used in step (iii) corresponds to +/- 1%, 2%, 3%, 4%, 5%, 7%, 8%, 9%, 10%, 12%, 15%, 17%, or 20%, the threshold value provided in table 1 for the miRNA or combination of miRNAs whose expression level is determined in step (i), particularly when the expression level(s) determined in step (i) is/are provided in counts per million.
42- The method according to any one of numbered embodiments 1-39, wherein the reference value used in step (iii) is provided as a range of value that corresponds to the range of threshold values indicated in table 1 for the miRNA or combination of miRNAs whose expression level is determined in step (i), particularly when the expression level(s) determined in step (i) is/are provided in CPM.
43- The method according to any one of numbered embodiments 1-39, wherein the reference value used in step (iii) is provided as a range of value, wherein the lower and upper values of the range correspond to +/-1%, 2%, 3%, 4%, 5%, 7%, 8%, 9%, 10%, 12%, 15%, 17%, or 20% the lower and upper values, respectively, of the range of threshold values provided in table 1 for the miRNA or combination of miRNAs whose expression level is determined in step (i), particularly when the expression level(s) determined in step (i) is/are provided in CPM.
44- The method according to any one numbered embodiments 1-39, wherein the reference value used in step (iii) corresponds to the threshold value provided in table 2 for the miRNA or combination of miRNAs whose expression level is determined in step (i), particularly when the expression level(s) determined in step (i) is/are provided in counts per million.
45- The method according to any one of numbered embodiments 1-39, wherein the reference value used in step (iii) corresponds to +/- 1%, 2%, 3%, 4%, 5%, 7%, 8%, 9%, 10%, 12%, 15%, 17%, or 20%, the threshold value provided in table 2 for the miRNA or combination of miRNAs whose expression level is determined in step (i), particularly when the expression level(s) determined in step (i) is/are provided in counts per million.
46- The method according to any one of numbered embodiments 1-39, wherein the reference value used in step (iii) is provided as a range of value that corresponds to the range of threshold values indicated in table 2 for the miRNA or combination of miRNAs whose expression level is determined in step (i), particularly when the expression level(s) determined in step (i) is/are provided in CPM.
47- The method according to any one of numbered embodiments 1-39, wherein the reference value used in step (iii) is provided as a range of value, wherein the lower and upper values of the range correspond to +/-1%, 2%, 3%, 4%, 5%, 7%, 8%, 9%, 10%, 12%, 15%, 17%, or 20% the lower and upper values, respectively, of the range of threshold values provided in table 2 for the miRNA or combination of miRNAs whose expression level is determined in step (i), particularly when the expression level(s) determined in step (i) is/are provided in CPM.
48- Use of at least one miRNA selected from the group consisting of miR-122-5p, miR-2110, miR-217-5p, miR-140-5p, miR-140-3p, and combinations thereof, as a biomarker for the diagnosis of cerebellar atrophy; or as a biomarker for deciding or recommending whether to initiate a therapeutic intervention in an individual suspicious of having cerebellar atrophy; or as a biomarker for determining the efficacy of a therapeutic intervention in a patient already diagnosed with cerebellar atrophy; or as a biomarker for classifying a subject into a cohort of subjects with high probabilities of having cerebellar atrophy or of subjects with low probabilities of having cerebellar atrophy.
49- Use of a kit for the diagnosis of cerebellar atrophy in an individual; or for deciding or recommending whether to initiate a therapeutic intervention in an individual suspicious of having cerebellar atrophy; or for determining the efficacy of a therapeutic intervention in a patient already diagnosed with cerebellar atrophy; or for classifying a subject into a subject cohort of subjects with high probabilities of having cerebellar atrophy or of subjects with low probabilities of having cerebellar atrophy; the kit comprising means for detecting the level of expression of at least one miRNA selected from the list consisting of miR-122-5p, miR-2110, miR-140-5p, miR-217-5p, miR-140-3p, and combinations thereof, and optionally a solid support.
50- The use according to numbered embodiment 49 , wherein the means for detecting the expression level of the miRNAs are primers.
51- The use according to any one of numbered embodiments 48-50, wherein the at least one miRNA comprises miR-122-5p.
52- The use according to any one of numbered embodiments 48-51, wherein the at least one miRNA comprises miR-2110.
53- The use according to any one of numbered embodiments 48-52, wherein the at least one miRNA comprises miR-140-5p.
54- The use according to any one of numbered embodiments 48-53, wherein the at least one miRNA comprises miR-217-5p.
55- The use according to any one of numbered embodiments 48-54, wherein the at least one miRNA comprises miR-140-3p.
56- The method according to any one of numbered embodiments 1-47 or the use according to any one of numbered embodiments 48 -55, wherein cerebellar atrophy is caused by or associated with a Phosphomannomutase 2 Congenital Disorder of Glycosylation, a cerebellar hemorrhage in a premature infant, CACNA1G protein dysfunction, CACNA1A protein dysfunction, CASK protein dysfunction, PPP2R1A protein dysfunction or combinations thereof.
57- The method according to any one of numbered embodiments 1-47 or the use according to any one of numbered embodiments 48-55, wherein cerebellar atrophy is caused by or associated with a Phosphomannomutase 2 Congenital Disorder of Glycosylation.
58- The method according to any one of numbered embodiments 1-47 or the use according to any one of numbered embodiments 48-55, wherein cerebellar atrophy is caused by or associated with a cerebellar hemorrhage in a premature infant, CACNA1G protein dysfunction, CACNA1A protein dysfunction, CASK protein dysfunction, PPP2R1A protein dysfunction or combinations thereof, particularly wherein CA is not caused by or associated with a Phosphomannomutase 2 Congenital Disorder of Glycosylation.
59- The method according to any one of numbered embodiments 1-9, 30-47, or 56-57, wherein cerebellar atrophy is caused by or associated with a Phosphomannomutase 2 Congenital Disorder of Glycosylation and step (i) comprises determining the expression level of:
   - miR-122-5p,
   - miR-122-5p, miR-217-5p and miR-140-3p,
   - miR-122-5p, miR-217-5p, and miR-140-5p,
   - miR-122-5p, miR-217-5p, miR-140-5p and miR-140-3p, or
   - miR-122-5p, miR-217-5, miR-140-5p, miR_2110 and miR-140-3p.
60- The method according to any one of numbered embodiments 1-9 or 30-47, 56 or 58, wherein the cerebellar atrophy is not caused by or associated with a Phosphomannomutase, particularly is caused by or associated with a cerebellar hemorrhage in a premature infant, CACNA1G protein dysfunction, CACNA1A protein dysfunction, CASK protein dysfunction, and/or PPP2R1A protein dysfunction, and step (i) comprises determining the expression level of:
   - miR_2110 and miR-140-5p,
   - miR_2110 and miR-217-5p,
   - miR_2110, miR-140-5p and miR-217-5p, or
   - miR-122-5p, miR-217-5, miR-140-5p, miR_2110 and miR-140-3p.
61- The use according to any one of numbered embodiments 48-58, wherein the cerebellar atrophy is caused by or associated with a Phosphomannomutase 2 Congenital Disorder of Glycosylation and the at least one miRNA selected from the group consisting of miR-122-5p, miR-2110, miR-140-5p, miR-217-5p, miR-140-3p, and combinations thereof corresponds to the miRNA or combination of miRNAs whose expression level is determined in step (i) of numbered embodiments 10-19, 28 or 59
62- The use according to any one of numbered embodiments 48-58, wherein the cerebellar atrophy is not caused by or associated with PMM2-CDG, particularly is caused by or associated with a cerebellar hemorrhage in a premature infant, CACNA1G protein dysfunction, CACNA1A protein dysfunction, CASK protein dysfunction, and/or PPP2R1A protein dysfunction and the at least one miRNA selected from the group consisting of miR-122-5p, miR-2110, miR-140-5p, miR-217-5p, miR-140-3p, and combinations thereof corresponds to the miRNA or combination of miRNAs whose expression level is determined in step (i) of numbered embodiments 20-28 or 60.
63- The method or the use according to any one of numbered embodiments 56, 58, 60 or 62, wherein the cerebellar atrophy is caused by or associated with CACNA1G protein dysfunction.
64- The method or the use according to any one of numbered embodiments 56, 58, 60 or 62, wherein the cerebellar atrophy is caused by or associated with CACNA1A protein dysfunction.
65- The method or the use according to any one of numbered embodiments 56, 58, 60 or 62, wherein the cerebellar atrophy is caused by or associated with CASK protein dysfunction.
66- The method or the use according to any one of numbered embodiments 56, 58, 60 or 62, wherein the cerebellar atrophy is caused by or associated with PPP2R1A protein dysfunction.
67- The method or the use according to any one of numbered embodiments 56, 58, 60, 62 or 63, wherein cerebellar atrophy is caused by or associated with CACNA1G protein dysfunction and consists of a CA caused by or associated with a mutation in the CACNA1G protein encoding gene of the subject, particularly selected from any of those indicated in the first aspect of the invention.
68- The method or the use according to any one of numbered embodiments 56, 58, 60, 62 or 64 wherein cerebellar atrophy is caused by or associated with CACNA1A protein dysfunction and consists of a CA caused by or associated with a mutation in CACNA1A protein encoding gene of the subject, particularly selected from any of those indicated in the first aspect of the invention.
69- The method or the use according to any one of numbered embodiments 56, 58, 60, 62, or 65, wherein cerebellar atrophy is caused by or associated with CASK protein dysfunction and consists of a CA caused by or associated with a mutation in CASK protein encoding gene of the subject, particularly selected from any of those indicated in the first aspect of the invention.
70- The method or the use according to any one of numbered embodiments 56, 58, 60, 62 or 66, wherein cerebellar atrophy is caused by or associated with PPP2R1A protein dysfunction and consists of a CA caused by or associated with a mutation in PPP2R1A protein encoding gene of the subject, particularly selected from any of those indicated in the first aspect of the invention.
71- The method or the use according to any one of numbered embodiments 56, 59, or 61, wherein cerebellar atrophy is caused by or associated with PMM2_CDG, or PMM2 protein dysfunction, and consists of a CA caused by or associated with a mutation in PMM2 protein encoding gene of the subject, particularly selected from any of those indicated in the first aspect of the invention.

In a particular embodiment of the invention, the CA referred in the methods that comprise step (i) as defined in numbered embodiments 10-19 is a cerebellar atrophy caused by or associated with PMM2-CDG.

In another particular embodiment of the invention, the CA referred in the methods that comprise step (i) as defined in numbered embodiments 20-27 is a cerebellar atrophy not caused by or associated with PMM2-CDG. More particularly, the CA referred in the methods that comprise step (i) as defined in clauses 20-27 is a cerebellar atrophy caused by or associated with a cerebellar hemorrhage in a premature infant, CACNA1G protein dysfunction, CACNA1A protein dysfunction, CASK protein dysfunction, PPP2R1A protein dysfunction or combinations thereof. The terms CACNA1G protein dysfunction, CACNA1A protein dysfunction, CASK protein dysfunction, PPP2R1A protein dysfunction have been defined in the first aspect of the invention and apply herein.

The following examples are provided by way of illustration, and they are not intended to be limiting of the present invention. Furthermore, the present invention covers all possible combinations of particular and preferred embodiments described herein.

### Examples

### Example 1: Materials and methods

Intravenous blood was obtained from 28 PMM2-CDG patients, 14 patients with non-PMM2-CDG CA and 67 controls. For all donors, 2 ml of whole blood samples were collected in EDTA anticoagulant tubes and inverted 10 times to ensure the mixing of anticoagulant with blood. Plasma isolation was done within 3 hours after blood collection. Haemolysis assessment was performed as described by Kirschner et al. (Kirschner et al. 2011). Samples with an A414 higher than 0.3AU were discarded, as described by Shah et al. (Shah, Soon, and Marsh 2016). The A414 refers to the absorbance level at 414 nm, measured by spectral analysis, which is indicative of free haemoglobin. Some exceptions were samples from icteric patients, where there is an increase in bilirubin, which has a high absorbance between 340 nm and 500 nm wavelengths and therefore affects the measurement (ArulVijayaVani, Mohanraj, and Reeta 2022). These samples were not discarded and hemolysis quality control (QC) was performed at the RNA level.

300 µl of plasma samples were processed using the miRNeasy Serum/Plasma Kit (Qiagen), following the manufacturer's protocol, increasing the volume of all the reagents by 50%, to account for the 50% increase in starting material. miRNA concentration was determined on Qubit 3.0 Fluorometer using a microRNA assay kit (Thermo Fisher Scientific) according to manufacturer's instructions. The size distribution of RNA fragments was assessed using RNA 6000 Nano Kit (Agilent) on a 2100 Bioanalyzer system. The levels of let-7b, miR-451 and miR-23a were quantified by RT-qPCR using a TaqMan MicroRNA Reverse Transcription Kit and the TaqMan Universal Master Mix II, no UNG (Thermo Fisher Scientific). The reactions were prepared as specified by the manufacturer. The abundance of let-7b was used to assess the concentration of a miRNA that is abundantly expressed in plasma. The abundance of miR-451 and miR-23a were used to quantify the hemolysis level, as described by Blondal et al. (Blondal et al. 2013).

miRNA library preparation was performed using the QIAseq miRNA library kit (Qiagen), according to the manufacturer's instructions, starting with 4 µl of miRNA extraction and adding 1 µl of QlAseq miRNA Library QC Spike-Ins (Qiagen) at the corresponding concentration. After library preparation, library QC consisted of cDNA library quantification using the Qubit dsDNA HS kit (Thermo Fisher Scientific) according to manufacturer's instructions. Samples were sequenced on a NextSeq500 - 75 cycles (1x75bp), to a mean depth of 10M reads per sample.

The Unique Molecular Identifiers (UMIs) were extracted with UMI-tools with standard parameters, adding a fixed seed for more reproducible results. For more information on UMI-tools, see (Smith, Heger, and Sudbery 2017). The adapter, low-quality reads (q < 20) and reads less than 16 nt in length were trimmed with Trim Galore. For more information on Trim Galore, see (Krueger [2016] 2023). Bowtie2 was used to align the RNA-seq data to the miRBase release 22.1, with the addition of Qiagen spike-in sequences. No mismatches were accepted. For more information on Bowtie2, see (Langmead and Salzberg 2012), for more information on miRBase, see (Kozomara, Birgaoanu, and Griffiths-Jones 2019). The gene count matrix was generated using SAMtools. For more information on SAMtools, see (Li et al. 2009).

Machine learning models were applied to obtain the genes associated with CA, using the R package caret . 10-fold cross-validation was repeated 10 times to determine the hyperparameters based on the F1 score, based on the training cohort. For more information on Caret, see (Kuhn 2008).

To determine the patient dependent score, the CPM normalized expression of the genes was combined through addition or subtraction (determined by sign in front of the gene -/+ in tables 1 and 2 below). The optimal threshold, the ROC curves and the AUCs were calculated by applying the roc function of the pROC package to the patient dependent scores in non-CA patients and CA patients having PMM2-CDG (see table 1 below) or in non-CA patients and CA patients not having PMM2-CDG (see table 2 below). For more information on the pROC package, see (Robin et al. 2011). If the patient dependent score value was below the threshold, then the sample was considered to be from a non-CA patient while if the value was above said threshold then the sample was considered to be from a CA patient. The threshold range was defined as the range between the two threshold-defining samples, being the sample classified as having CA with the highest score and the sample classified as control with the lowest score.

### Example 2. Results

From the machine learning model, a total of 5 miRNAs were found that could be used to discriminate between CA and controls: miR-122-5p, miR-2110, miR-217-5p, miR-140-3p and miR-140-5p.

Using the miRNAs selected by the machine learning model, several miRNA combinations were obtained with high discriminative value, evidenced by the high AUC in several of the gene combinations in Tables 1 and 2.

In the comparison between non-CA patients and CA patients having PMM2-CDG (see table 1 below), we can find one miRNA, miR-122-5p, which has a high AUC by itself but an even increased AUC when combined with other genes, being especially relevant the inclusion of the miRNAs miR-217-5p and miR-140-5p.

In the comparison between non-CA patients and CA patients not having PMM2-CDG (see table 2 below), we can find one miRNA, miR-2110, which has a high AUC by itself but an even increased AUC when combined with other genes, being especially relevant the inclusion of the miRNAs miR-217-5p and miR-140-5p.

**Table 1. AUCs, thresholds and threshold ranges of each of the miRNA or combination of miRNAs, obtained from the analysis of samples of non-CA patients and CA patients having PMM2-CDG.**

| **miRNA_combination** | **AUC** | **Threshold** | **Threshold-range** |
|---|---|---|---|
| miR-122-5p | 0.76 | -0.084 | -0.093,-0.074 |
| -(miR-2110) | 0.631 | 0.187 | 0.184,0.191 |
| miR-217-5p | 0.629 | 0.136 | -0.266,0.539 |
| -(miR-140-3p) | 0.691 | 0.445 | 0.441,0.449 |
| -(miR-140-5p) | 0.676 | 0.119 | 0.118,0.12 |
| miR-122-5p-(miR-2110) | 0.735 | -0.149 | -0.15,-0.148 |
| miR-122-5p+miR-217-5p | 0.824 | -0.35 | -0.359,-0.34 |
| miR-122-5p-(miR-140-3p) | 0.809 | 0.37 | 0.36,0.379 |
| miR-122-5p-(miR-140-5p) | 0.841 | 0.274 | 0.272,0.277 |
| miR-217-5p-(miR-2110) | 0.708 | 0.724 | 0.706,0.742 |
| -(miR-2110)-(miR-140-3p) | 0.717 | 0.068 | 0.056,0.08 |
| -(miR-2110)-(miR-140-5p) | 0.727 | 0.748 | 0.745,0.751 |
| miR-217-5p-(miR-140-3p) | 0.78 | 0.32 | 0.3,0.34 |
| miR-217-5p-(miR-140-5p) | 0.759 | -0.147 | -0.148,-0.146 |
| -(miR-140-3p)-(miR-140-5p) | 0.704 | 0.926 | 0.926,0.927 |
| miR-122-5p-(miR-2110)+miR-217-5p | 0.774 | 0.684 | 0.622,0.745 |
| miR-122-5p-(miR-2110)-(miR-140-3p) | 0.805 | 0.317 | 0.309,0.325 |
| miR-122-5p-(miR-2110)-(miR-140-5p) | 0.826 | 0.432 | 0.43,0.434 |
| miR-122-5p+miR-217-5p-(miR-140-3p) | 0.858 | 0.214 | 0.195,0.234 |
| miR-122-5p+miR-217-5p-(miR-140-5p) | 0.873 | 0.008 | 0.006,0.011 |
| miR-122-5p-(miR-140-3p)-(miR-140-5p) | 0.81 | 0.25 | 0.239,0.262 |
| miR-217-5p-(miR-2110)-(miR-140-3p) | 0.777 | 0.501 | 0.496,0.506 |
| miR-217-5p-(miR-2110)-(miR-140-5p) | 0.783 | 0.482 | 0.479,0.485 |
| -(miR-2110)-(miR-140-3p)-(miR-140-5p) | 0.734 | 0.26 | 0.131,0.389 |
| miR-217-5p-(miR-140-3p)-(miR-140-5p) | 0.782 | 0.66 | 0.66,0.661 |
| miR-122-5p-(miR-2110)+miR-217-5p-(miR-140-3p) | 0.838 | 0.051 | 0.043,0.059 |
| miR-122-5p-(miR-2110)+miR-217-5p-(miR-140-5p) | 0.837 | 0.166 | 0.164,0.168 |
| miR-122-5p-(miR-2110)-(miR-140-3p)-(miR-140-5p) | 0.827 | 0.845 | 0.807,0.882 |
| miR-122-5p+miR-217-5p-(miR-140-3p)-(miR-140-5p) | 0.854 | -0.016 | -0.027,-0.004 |
| miR-217-5p-(miR-2110)-(miR-140-3p)-(miR-140-5p) | 0.796 | 0.59 | 0.565,0.614 |
| miR-122-5p-(miR-2110)+miR-217-5p-(miR-140-3p)-(miR-140-5p) | 0.855 | 0.58 | 0.541,0.619 |

**Table 2. AUCs, thresholds and threshold ranges of each of the miRNA or combination of miRNAs, obtained from the analysis of samples of non-CA patients and CA patients not having PMM2-CDG.**

| **miRNA_combination** | **AUC** | **Threshold** | **Threshold_range** |
|---|---|---|---|
| miR-122-5p | 0.587 | -0.425 | -0.433,-0.418 |
| -(miR-2110) | 0.695 | 0.339 | 0.336,0.341 |
| miR-217-5p | 0.528 | 0.886 | -0.133,1.904 |
| -(miR-140-3p) | 0.541 | 0.588 | 0.565,0.611 |
| -(miR-140-5p) | 0.671 | 0.203 | 0.197,0.209 |
| miR-122-5p-(miR-2110) | 0.589 | 0.104 | 0.075,0.132 |
| miR-122-5p+miR-217-5p | 0.57 | -0.558 | -0.565,-0.551 |
| miR-122-5p-(miR-140-3p) | 0.564 | -0.025 | -0.041,-0.01 |
| miR-122-5p-(miR-140-5p) | 0.535 | -0.942 | -0.978,-0.905 |
| miR-217-5p-(miR-2110) | 0.7 | 0.206 | 0.204,0.209 |
| -(miR-2110)-(miR-140-3p) | 0.664 | 0.996 | 0.946,1.045 |
| -(miR-2110)-(miR-140-5p) | 0.708 | 1.031 | 1.024,1.038 |
| miR-217-5p-(miR-140-3p) | 0.582 | 0.455 | 0.433,0.478 |
| miR-217-5p-(miR-140-5p) | 0.688 | 0.551 | 0.55,0.551 |
| -(miR-140-3p)-(miR-140-5p) | 0.583 | 0.876 | 0.867,0.885 |
| miR-122-5p-(miR-2110)+miR-217-5p | 0.604 | -0.029 | -0.058,0 |
| miR-122-5p-(miR-2110)-(miR-140-3p) | 0.57 | 0.601 | 0.582,0.619 |
| miR-122-5p-(miR-2110)-(miR-140-5p) | 0.62 | 0.439 | 0.42,0.459 |
| miR-122-5p+miR-217-5p-(miR-140-3p) | 0.521 | -0.101 | -0.106,-0.095 |
| miR-122-5p+miR-217-5p-(miR-140-5p) | 0.562 | -1.074 | -1.111,-1.038 |
| miR-122-5p-(miR-140-3p)-(miR-140-5p) | 0.523 | 0.083 | 2.248,0.148 |
| miR-217-5p-(miR-2110)-(miR-140-3p) | 0.689 | 0.863 | 0.813,0.912 |
| miR-217-5p-(miR-2110)-(miR-140-5p) | 0.711 | 0.898 | 0.891,0.905 |
| -(miR-2110)-(miR-140-3p)-(miR-140-5p) | 0.669 | 1.371 | 1.35,1.393 |
| miR-217-5p-(miR-140-3p)-(miR-140-5p) | 0.611 | 0.743 | 0.734,0.752 |
| miR-122-5p-(miR-2110)+miR-217-5p-(miR-140-3p) | 0.596 | 0.468 | 0.449,0.486 |
| miR-122-5p-(miR-2110)+miR-217-5p-(miR-140-5p) | 0.633 | 0.307 | 0.287,0.326 |
| miR-122-5p-(miR-2110)-(miR-140-3p)-(miR-140-5p) | 0.6 | 0.902 | 0.859,0.945 |
| miR-122-5p+miR-217-5p-(miR-140-3p)-(miR-140-5p) | 0.553 | -0.05 | -0.064,-0.037 |
| miR-217-5p-(miR-2110)-(miR-140-3p)-(miR-140-5p) | 0.684 | 1.239 | 1.217,1.26 |
| miR-122-5p-(miR-2110)+miR-217-5p-(miR-140-3p)-(miR-140-5p) | 0.623 | 0.769 | 0.727,0.812 |

### Citation list

- ArulVijayaVani, Subramaniam, Palani Selvam Mohanraj, and Rajagambeeram Reeta. 2022. "Evaluating Interference of Lipemia on Routine Clinical Biochemical Tests." Journal of Laboratory Physicians 15 (2): 269-75. https://doi.org/10.1055/s-0042-1758664.
- Blondal, Thorarinn, Søren Jensby Nielsen, Adam Baker, Ditte Andreasen, Peter Mouritzen, Maria Wrang Teilum, and Ina K. Dahlsveen. 2013. "Assessing Sample and miRNA Profile Quality in Serum and Plasma or Other Biofluids." Methods, Transcriptional Biomarkers, 59 (1): S1-6. https://doi.org/10.1016/j.ymeth.2012.09.015.
- Kirschner, Michaela B., Steven C. Kao, J. James Edelman, Nicola J. Armstrong, Michael P. Vallely, Nico van Zandwijk, and Glen Reid. 2011. "Haemolysis during Sample Preparation Alters microRNA Content of Plasma." PLOS ONE 6 (9): e24145. https://doi.org/10.1371/journal.pone.0024145.
- Kozomara, Ana, Maria Birgaoanu, and Sam Griffiths-Jones. 2019. "miRBase: From microRNA Sequences to Function." Nucleic Acids Research 47 (D1): D155-62. https://doi.org/10.1093/nar/gky1141.
- Krueger, Felix.(2016) 2023. "Trim Galore." Perl.https://github.com/FelixKrueger/TrimGalore. Kuhn, Max. 2008. "Building Predictive Models in R Using the Caret Package." Journal of Statistical Software 28 (November):1-26. https://doi.org/10.18637/jss.v028.i05.
- Langmead, Ben, and Steven L Salzberg. 2012. "Fast Gapped-Read Alignment with Bowtie 2." Nature Methods 9 (4): 357-59. https://doi.org/10.1038/nmeth.1923.
- Li, Heng, Bob Handsaker, Alec Wysoker, Tim Fennell, Jue Ruan, Nils Homer, Gabor Marth, Goncalo Abecasis, Richard Durbin, and 1000 Genome Project Data Processing Subgroup. 2009. "The Sequence Alignment/Map Format and SAMtools." Bioinformatics 25 (16): 2078-79. https://doi.org/10.1093/bioinformatics/btp352.
- Robin, Xavier, Natacha Turck, Alexandre Hainard, Natalia Tiberti, Frédérique Lisacek, Jean-Charles Sanchez, and Markus Müller. 2011. "pROC: An Open-Source Package for R and S+ to Analyze and Compare ROC Curves." BMC Bioinformatics 12 (1): 77. https://doi.org/10.1186/1471-2105-12-77.
- Shah, Jaynish S., Patsy S. Soon, and Deborah J. Marsh. 2016. "Comparison of Methodologies to Detect Low Levels of Hemolysis in Serum for Accurate Assessment of Serum microRNAs." PLoS ONE 11 (4): e0153200. https://doi.org/10.1371/journal.pone.0153200.
- Smith, Tom Sean, Andreas Heger, and lan Sudbery. 2017. "UMI-Tools: Modelling Sequencing Errors in Unique Molecular Identifiers to Improve Quantification Accuracy." Genome Research, January, gr.209601.116. https://doi.org/10.1101/gr.209601.116.

## Claims

1. An *in vitro* method for the diagnosis of cerebellar atrophy in a subject comprising:
(i) determining, in a sample isolated from the subject, the expression level of at least one miRNA selected from the group consisting of miR-122-5p, miR-2110, miR-140-5p, miR-217-5p, miR-140-3p, and combinations thereof,
(ii) calculating a variable S, wherein: $S = A - B - C + D - E$ and wherein:
- A is the expression level of miR-122-5p determined in step (i), or 0 when the expression level of miR-122-5p is not determined in step (i),
- B is the expression level of miR-2110 determined in step (i), or 0 when the expression level of miR-2110 is not determined in step (i),
- C is the expression level of miR-140-5p determined in step (i), or 0 when the expression level of miR-140-5p is not determined in step (i),
- D is the expression level of miR-217-5p determined in step (i), or 0 when the expression level of miR-217-5p is not determined in step (i),
- E is the expression level of miR-140-3p determined in step (i), or 0 when the expression level of miR-140-3p is not determined in step (i), and
(iii) diagnosing the subject as having cerebellar atrophy if S calculated in step (ii) is equal or higher than a corresponding reference value and as not having cerebellar atrophy if S calculated in step (ii) is lower than the corresponding reference value.

2. The method according to claim 1, wherein step (i) further comprises determining in the sample isolated from the patient, the expression level of at least one additional miRNA and the variable S in step (ii) is: $S = A - B - C + D - E + {\sum }_{i = 1}^{N} {a}_{i} {F}_{i} ,$ wherein
F is the expression level of the at least one additional miRNA, N is the number of additional miRNAs whose expression level is determined in step (i) and a is (1) if an increased expression level of the corresponding miRNA is associated to having cerebellar atrophy or is (-1) if a decreased expression level of the corresponding miRNA is associated to having cerebellar atrophy.

3. The method according to any one of claims 1-2, wherein step (i) comprises determining in the isolated sample, the expression level of:
- miR-122-5p,
- miR-122-5p, miR-217-5p and miR-140-3p,
- miR-122-5p, miR-217-5p and miR-140-5p,
- miR-122-5p, miR-217-5p, miR-140-5p and miR-140-3p
- miR-2110 and miR-217-5p,
- miR-2110 and miR-140-5p,
- miR-2110, miR-140-5p and miR-217-5p, or
- miR-122-5p, miR-217-5, miR-140-5p, miR-2110 and miR-140-3p.

4. An *in vitro* method for deciding or recommending whether to initiate a therapeutic intervention in an individual suspicious of having cerebellar atrophy, wherein the method comprises steps (i)-(ii) as defined in any one of claims 1-3, and (iii) if S calculated in step (ii) is equal or higher than the corresponding reference value, it is indicative that the individual has to start a therapeutic intervention.

5. An *in vitro* method for determining the efficacy of a therapeutic intervention in a patient already diagnosed with cerebellar atrophy, wherein the method comprises performing steps (i)-(ii) as defined in any one of claims 1-3 before and after starting the therapeutic intervention, and if S calculated in step (ii) obtained after starting the therapeutic intervention, is lower than S calculated in step (ii) obtained before starting the therapeutic intervention, it is indicative that the therapeutic intervention is effective in the treatment of cerebellar atrophy.

6. A method for classifying a subject into a subject cohort, wherein the method comprises steps (i)-(ii) as defined in any one of claims 1-3 and (iii) classifying the subject into:
- a cohort of subjects with high probability of suffering cerebellar atrophy if S calculated in step (ii) is equal or higher than the corresponding reference value, or
- a cohort of subjects with low probability of suffering cerebellar atrophy if S calculated in step (ii) is lower than the corresponding reference value.

7. The method according to claim 6, wherein it is decided or recommended to perform a method for the diagnosis of cerebellar atrophy to the subject with high probability of having cerebellar atrophy.

8. The method according to any one of the previous claims wherein the sample is a blood sample, in particular, a plasma sample.

9. Use of at least one miRNA selected from the group consisting of miR-122-5p, miR-2110, miR-217-5p, miR-140-5p, miR-140-3p, and combinations thereof, as a biomarker for the diagnosis of cerebellar atrophy; or as a biomarker for deciding or recommending whether to initiate a therapeutic intervention in an individual suspicious of having cerebellar atrophy; or as a biomarker for determining the efficacy of a therapeutic intervention in a patient already diagnosed with cerebellar atrophy; or as a biomarker for classifying a subject into a cohort of subjects with high probabilities of having cerebellar atrophy or of subjects with low probabilities of having cerebellar atrophy.

10. Use of a kit for the diagnosis of cerebellar atrophy in an individual; or for deciding or recommending whether to initiate a therapeutic intervention in an individual suspicious of having cerebellar atrophy; or for determining the efficacy of a therapeutic intervention in a patient already diagnosed with cerebellar atrophy; or for classifying a subject into a cohort of subjects with high probabilities of having cerebellar atrophy or of subjects with low probabilities of having cerebellar atrophy; the kit comprising means for detecting the level of expression of at least one miRNA selected from the list consisting of miR-122-5p, miR-2110, miR-140-5p, miR-217-5p, miR-140-3p, and combinations thereof, and optionally a solid support.

11. The method according to any one of claims 1-8 or the use according to any one of claims 9-10, wherein cerebellar atrophy is caused by or associated with a Phosphomannomutase 2 Congenital Disorder of Glycosylation, a cerebellar hemorrhage in a premature infant, CACNA1G protein dysfunction, CACNA1A protein dysfunction, CASK protein dysfunction, and/or PPP2R1A protein dysfunction, particularly is caused by or associated with a Phosphomannomutase 2 Congenital Disorder of Glycosylation.

12. The method according to any one of claims 1-8 or 11, wherein cerebellar atrophy is caused by or associated with a Phosphomannomutase 2 Congenital Disorder of Glycosylation and step (i) comprises determining the expression level of:
- miR-122-5p,
- miR-122-5p, miR-217-5p and miR-140-3p,
- miR-122-5p, miR-217-5p, and miR-140-5p,
- miR-122-5p, miR-217-5p, miR-140-5p and miR-140-3p, or
- miR-122-5p, miR-217-5, miR-140-5p, miR_2110 and miR-140-3p.

13. The method according to any one of claims 1-8 or 11, wherein the cerebellar atrophy is not caused by or associated with a Phosphomannomutase 2 Congenital Disorder of Glycosylation , particularly is caused by or associated with cerebellar hemorrhage in a premature infant, CACNA1G protein dysfunction, CACNA1A protein dysfunction, CASK protein dysfunction, PPP2R1A protein dysfunction and combinations thereof, and step (i) comprises determining the expression level of:
- miR_2110 and miR-140-5p,
- miR_2110 and miR-217-5p,
- miR_2110, miR-140-5p and miR-217-5p, or
- miR-122-5p, miR-217-5, miR-140-5p, miR_2110 and miR-140-3p.

14. The use according to any one of claims 9-11, wherein the cerebellar atrophy is caused by or associated with a Phosphomannomutase 2 Congenital Disorder of Glycosylation and the at least one miRNA selected from the group consisting of miR-122-5p, miR-2110, miR-140-5p, miR-217-5p, miR-140-3p, and combinations thereof is:
- miR-122-5p,
- miR-122-5p, miR-217-5p and miR-140-3p,
- miR-122-5p, miR-217-5p, miR-140-5p and miR-140-3p, or
- miR-122-5p, miR-217-5, miR-140-5p, miR_2110 and miR-140-3p.

15. The use according to any one of the claims 9-11, wherein the cerebellar atrophy is not caused by or associated with Phosphomannomutase 2 Congenital Disorder of Glycosylation, particularly is caused by or associated with a cerebellar hemorrhage in a premature infant, CACNA1G protein dysfunction, CACNA1A protein dysfunction, CASK protein dysfunction, PPP2R1A protein dysfunction and combinations thereof, and the at least one miRNA selected from the group consisting of miR-122-5p, miR-2110, miR-140-5p, miR-217-5p, miR-140-3p, and combinations thereof is:
- miR_2110 and miR-140-5p,
- miR_2110 and miR-217-5p,
- miR_2110, miR-140-5p and miR-217-5p, or
- miR-122-5p, miR-217-5, miR-140-5p, miR_2110 and miR-140-3p.
